(19) Europäisches Patentamt / European Patent Office / Office européen des brevets

(11) EP 2 704 727 B1

(12) **EUROPEAN PATENT SPECIFICATION**

(45) Date of publication and mention of the grant of the patent:
**12.02.2020 Bulletin 2020/07**

(21) Application number: **12786297.7**

(22) Date of filing: **24.04.2012**

(51) Int Cl.:
*A61K 31/738* (2006.01)      *A61K 47/30* (2006.01)
*A61K 9/06* (2006.01)       *A61F 2/02* (2006.01)
*A61P 17/00* (2006.01)      *A61L 27/20* (2006.01)
*A61L 27/52* (2006.01)      *A61L 27/54* (2006.01)
*A61K 38/18* (2006.01)

(86) International application number:
**PCT/US2012/034802**

(87) International publication number:
**WO 2012/158312 (22.11.2012 Gazette 2012/47)**

(54) **SKIN AND HAIR REGENERATION USING POLYSACCHARIDE-BASED HYDROGELS**

HAUT UND HAARREGENERATION MIT HYDROGELEN AUF POLYSACCHARIDBASIS

RÉGÉNÉRATION CUTANÉE ET PILEUSE FAISANT APPEL À DES HYDROGELS À BASE DE POLYSACCHARIDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.05.2011 US 201161483518 P**
**28.11.2011 US 201161563954 P**

(43) Date of publication of application:
**12.03.2014 Bulletin 2014/11**

(73) Proprietor: **The Johns Hopkins University Baltimore, MD 21218 (US)**

(72) Inventors:
• **SUN, Guoming**
 **Baltimore**
 **Maryland 21218 (US)**
• **ZHANG, Xianjie**
 **Baltimore**
 **Maryland 21218 (US)**
• **HARMON, John W.**
 **Baltimore**
 **Maryland 21224 (US)**
• **GERECHT, Sharon**
 **Baltimore**
 **Maryland 21218 (US)**

(74) Representative: **Appleyard Lees IP LLP**
 **15 Clare Road**
 **Halifax HX1 2HY (GB)**

(56) References cited:
WO-A2-2010/078036      WO-A2-2011/060095
WO-A2-2012/003370

• SUN G ET AL: "Functional neovascularization of biodegradable dextran hydrogels with multiple angiogenic growth factors", BIOMATERIALS, ELSEVIER SCIENCE PUBLISHERS BV., BARKING, GB, vol. 32, no. 1, 1 January 2011 (2011-01-01), pages 95-106, XP027493693, ISSN: 0142-9612, DOI: 10.1016/J.BIOMATERIALS.2010.08.091 [retrieved on 2010-09-25]
• GUOMING SUN ET AL: "Functional groups affect physical and biological properties of dextran-based hydrogels", JOURNAL OF BIOMEDICAL MATERIALS RESEARCH PART A, vol. 9999A, 1 January 2009 (2009-01-01), pages NA-NA, XP055005572, ISSN: 1549-3296, DOI: 10.1002/jbm.a.32604
• G. SUN ET AL: "Dextran hydrogel scaffolds enhance angiogenic responses and promote complete skin regeneration during burn wound healing", PROCEEDINGS OF THE NATIONAL ACADEMY OF SCIENCES, vol. 108, no. 52, 27 December 2011 (2011-12-27), pages 20976-20981, XP055159511, ISSN: 0027-8424, DOI: 10.1073/pnas.1115973108

**Description**

**CROSS-REFERENCE TO RELATED APPLICATION**

This application claims priority to U.S. Provisional Application No. 61/483,518 filed May 6, 2011 and U.S. Provisional Application No. 61/563,954 filed November 28, 2011.

This research was partially funded by NIH grant R01HL107938. The Government has certain rights in this invention.

**BACKGROUND**

FIELD OF THE INVENTION

The present invention is related to skin and hair regeneration after injury using biocompatible polysaccharide-based hydrogels.

BACKGROUND OF THE INVENTION

Polymeric hydrogels have found a broad range of pharmaceutical and biomedical applications due to their three-dimensional structural and their functional similarity to natural tissues. A wide variety of hydrogels have been prepared, based on either physical or chemical crosslinking methods. The chemical crosslinking approach to designing biodegradable hydrogels is desirable because they are relatively easy to formulate by controlling experimental parameters, such as the type and concentration of crosslinking agents, initiator concentrations, and the ratios and concentrations of precursors.

Burn injuries constitute a major worldwide public health problem (Zhang et al., Arch Surg, vol. 145, no. 3, pp. 259-266, 2010) and cause more severe physiological stress than other traumas (Sen et al., J. Burn Care Res., vol. 31, no. 6, pp. 836-848, 2010; Fagenholz et al., J Burn Care Res, vol. 28, no. 5, pp. 681-690, 2007). Superficial burns usually heal with minimal scarring, but treatments for second- and third-degree burn injuries remain far from optimal (Zhang et al., Arch Surg, vol. 145, no. 3, pp. 259-266, 2010; Zhang et al., Wound Repair Regen., vol. 18, no. 2, pp. 193-201, 2010). Burn-induced full thickness skin injuries result in rapid and dangerous liquid loss and impair many vital functions that skin performs. The healing process for adult skin wounds is complex, requiring the collaborative efforts of various tissues and cell lineages, as well as both extracellular and intracellular signals (Kirker et al., Biomaterials, vol. 23, no. 17, pp. 3661-3671, 2002; Gurtner et al., Nature, vol. 453, no. 7193, pp. 314-321, 2008). Although research has elucidated many details of the basic wound healing process (Li et al., Microscopy Research and Technique, vol. 60, no. 1, 107-114, 2003), the regeneration of perfect skin remains an elusive goal (Martin, Science, vol. 276, no. 5309, pp. 75-81, 1997).

Third-degree burns involve damage to both epidermal and dermal layers and may also cause damage to underlying muscles, bones, and tendons. Such burns heal with thick scars, resulting in contractures that distort the surrounding tissue. Deep third-degree burns usually require skin grafting to achieve wound closure, but the cosmetic and functional results are less than optimal, as the grafted skin is thin and vulnerable to re-injury. In general, wound repair has three classic stages: the inflammatory, proliferative, and remodeling stages (Gurtner et al., Nature, vol. 453, no. 7193, pp. 314-321, 2008; Tibbs, Radiotherapy and Oncology, vol. 42, no. 2, pp. 99-106, 1997; Haroon et al., The FASEB Journal, vol. 13, no. 13, pp. 1787-1795, 1999). The inflammatory stage begins with hemostasis and formation of the platelet plug. Platelets release growth factors to attract neutrophils and macrophages (Steed, Surgical Clinics of North America, vol. 77, no. 3, pp. 575-586, 1997). Neutrophil influx, an early inflammatory response, is essential for the clearance of bacteria and both cellular and foreign debris (Kim et al., J Invest Dermatol, vol. 128, no. 7, pp. 1812-1820, 2008), while macrophages produce growth factors that induce and accelerate angiogenesis during wound healing (Greenhalgh, The International Journal of Biochemistry & Cell Biology, vol. 30, no. 9, pp. 1019-1030, 1998). The inflammatory stage overlaps with the proliferative stage where an eschar forms on the surface of the wound. In the proliferative stage, most cells from the inflammatory stage of repair have disappeared from the wound, and new blood vessels now populate the area (Gurtner et al., Nature, vol. 453, no. 7193, pp. 314-321, 2008). Initiation of the remodeling stage occurs when collagen formation and breakdown reach a state of equilibrium. In this stage, fibroblasts that have migrated into the wound lay down disorganized collagen, and fibroblasts differentiate into myofibroblasts, causing tissue contraction. Collagen reorganizes along lines of tension and crosslinks, giving additional strength. Nevertheless, wounds are unable to attain the same mechanical strength as uninjured skin (Singer et al., New England Journal of Medicine, vol. 341, no. 10, pp. 738-746, 1999).

Angiogenesis and neovascularization are critical determinants of the wound-healing outcomes for deep burn injuries (Tredget, Journal of Trauma-Injury Infection and Critical Care, vol. 62, no. 6, pp. S69-S69, 2007). Severe burn wounds lose more dermal blood flow than superficial burns. Newly formed blood vessels participate in the healing process, providing nutrition and oxygen to growing tissues (Li et al., Microscopy Research and Technique, vol. 60, no. 1, 107-114, 2003). The repair of the dermal vasculature largely determines whether second-degree burns heal promptly and primarily or, due to delayed healing, they become third-degree burns, with the consequent necrosis and damaging scarring. Thus, encouraging angiogenesis could promote dermal layer regeneration and complete skin formation. Hydrogels, structurally similar to the natural extracellular matrix (ECM), can be designed to provide an instructive environment for the three-dimensional (3D) assembly of vascular networks. Many studies of hydrogel-based scaffolds have focused on applications in healing wounds (Kirker et al., Biomaterials, vol. 23, no. 17, pp. 3661-3671, 2002; Boucard et al. Biomaterials, vol. 28, no. 24, pp. 3478-3488, 2007; Kiyozumi et al., Burns, vol. 33, no. 5, pp. 642-648, 2007; Kim et al., Biomaterials, vol. 30, no. 22, pp. 3742-3748, 2009; Madsen et al., Biomacromolecules, vol. 9, no. 8, pp. 2265-2275, 2008; Shepherd et al., Biomaterials, vol. 32, no. 1, pp. 258-267, 2011; Balakrishnan et al., Biomaterials, vol. 26, no. 32, pp. 6335-6342, 2005). Beyond their utility as scaffolds, hydrogels can also deliver cytokines and growth factors (Puolakkainen et al., Journal of Surgical Research, vol. 58, no. 3, pp. 321-329, 1995; Kiyozumi et al., Journal of Biomedical Materials Research Part B: Applied Biomaterials, vol. 79B, no. 1, pp. 129-136, 2006), antibiotics (Shepherd et al., Biomaterials, vol. 32, no. 1, pp. 258-267, 2011), and cells (Liu et al., Biomaterials, vol. 30, no. 8, pp. 1453-1461, 2009; Lee et al., Mol Ther, vol. 15, no. 6, pp. 1189-1194, 2007) to allow complete skin regeneration. In addition, WO 2010/078036 and WO 2012/003370 relate to biocompatible polysaccharide-based hydrogels for vascular regeneration. WO 2011/060095 relates to growth media for vascular linear cells using oligosaccharide-based hydrogels.

## SUMMARY

Embodiments of the invention include methods of promoting skin regeneration by topically administering to a subject with an area of full thickness skin injury damaging the skin, a hydrogel on at least a portion of the injured area. The hydrogel comprises a crosslinked composition as defined in claim 1.

Embodiments of the invention include methods of promoting hair follicle regeneration by topically administering to a subject with an area of full thickness skin injury damaging the skin, a hydrogel on the injured area. The hydrogel comprises a crosslinked composition as defined in claim 2.

Embodiments of the invention include methods of reducing scarring by topically administering to a subject with an area of full thickness skin injury damaging the skin, a hydrogel on the injured area. The hydrogel comprises a crosslinked composition as defined in claim 3.

In some of the above embodiments, $R^7$ is hydrogen, and the second crosslinkable molecule is poly(ethylene glycol) diacrylate. In some of the above embodiments, Z is $NR^4R^5$.

In some of the above embodiments, at least one hydroxyl-substituted saccharide monomer is a glucopyranose monomer. The polysaccharide is dextran. In some embodiments, the dextran has an average molecular weight of at least 20,000.

In some of the above embodiments, the second crosslinkable molecule is poly(ethylene glycol) diacrylate. In some embodiments, the poly(ethylene glycol) diacrylate has a molecular weight of at least 2000.

In some of the above embodiments, the polysaccharide further comprises a second substituted hydroxyl group having the formula (IV), where formula (III) and formula (IV) are different, and the substituted hydroxyl group of formula (III) and formula (IV) may be on the same or different monomers; wherein formula (IV) is $Y-(CR^2R^3)_n-Z$ where Y is $-O_1-$ or $-O_1C(O)-$, or $-O_1C(O)NR^1-$, $O_1$ is the oxygen atom of said substituted hydroxyl group, and $R^1$ is hydrogen or $C_1-C_4$ alkyl; n = 1, 2, 3, or 4; Z is selected from the group consisting of $-CO_2H$ or $NR^4R^5$, where $R^4$ and $R^5$ are independently hydrogen or $C_1-C_4$ alkyl; $R^2$ and $R^3$ are independently hydrogen, $C_1-C_4$ alkyl, or may combine to form a 3-6 membered ring, and when n>1, $R^2$ and $R^3$ on adjacent carbons may form a double or triple bond, or $R^2$ and $R^3$ on different carbon atoms may form a 3-6 membered ring.

In some of the above embodiments, the hydrogel further includes one or more of a protein, oligonucleotide or pharmaceutical agent. In some embodiments including a protein, the protein is a growth factor. In some embodiments, the growth factor is vascular endothelial growth factor (VEGF). In some embodiments including a pharmaceutical agent, the pharmaceutical agent is an antibiotic, antimicrobial, antibacterial, antifungal, or antiviral compound. In some embodiments, the photocrosslinked composition does not include a protein or growth factor when topically administered.

In some embodiments, the area of injury damaging the skin is a second degree burn or third degree burn. In some embodiments, the topical administration further involves placing the hydrogel to extend the hydrogel over an uninjured area. In some embodiments, topical administration involves covering the entire injured area with the hydrogel.

## BRIEF DESCRIPTION OF THE DRAWINGS

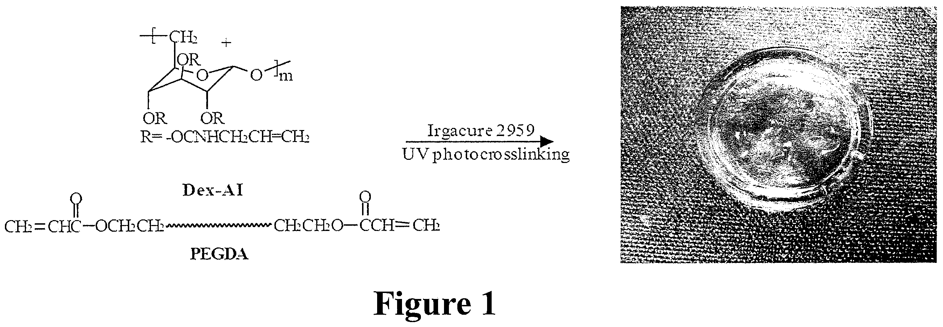

**Figure 1** shows hydrogel preparation.

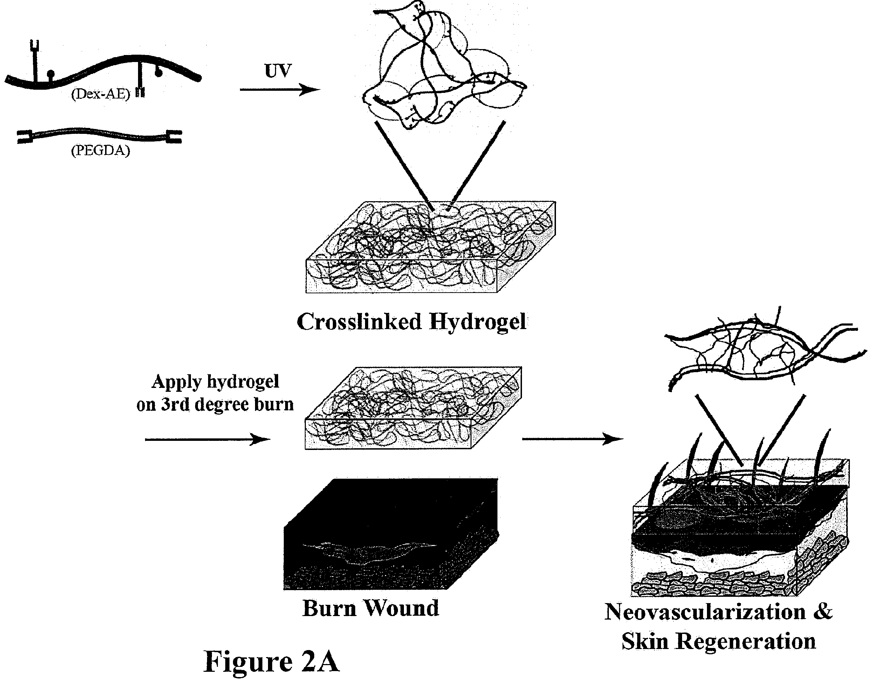

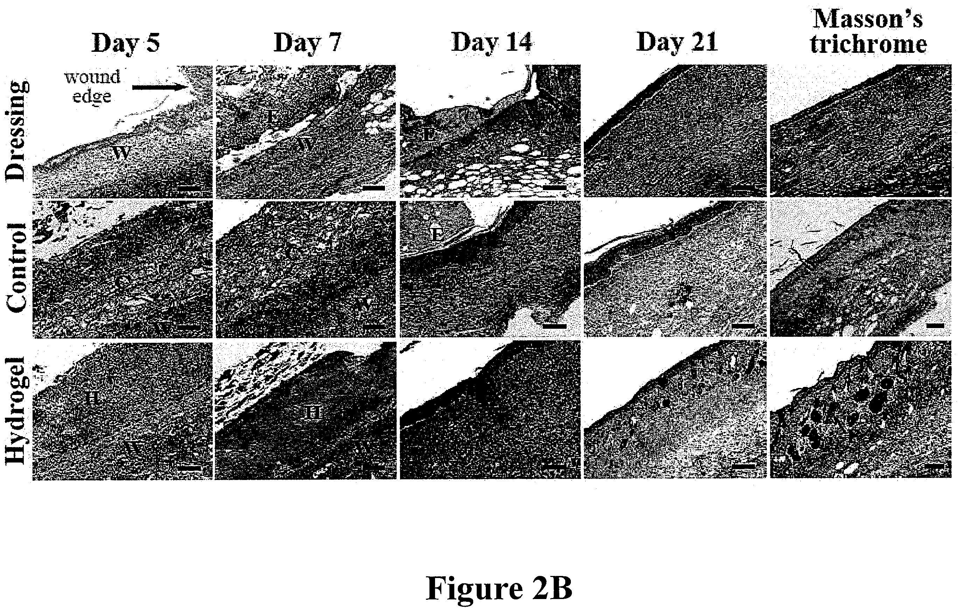

**Figure 2** shows dextran hydrogel as a therapeutic modality. **Figure 2A** shows dextran-based hydrogel promotes neovascularization: precise structure manipulation allows rapid, efficient, and functional neovascularization. **Figure 2B** shows representative images of H&E stained histological sections at time intervals show that dextran hydrogel promoted wound healing with complete skin appendage regeneration. Masson's trichrome staining indicates distinct collagen structures formed in dermal layer by day 21. Wound edge indicates the excision rim. W = Wound area, H = Hydrogel scaffold, C = Control scaffold, E-Eschar, F= Follicle, and S=sebaceous gland. Scale bars = 100 $\mu$m.

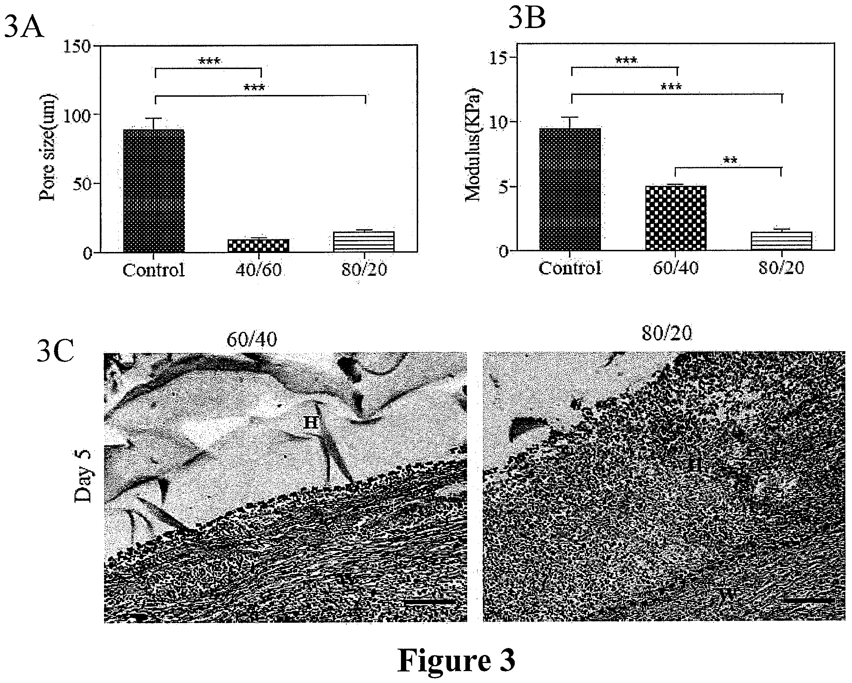

**Figure 3** shows characterization of scaffold treatments. Dextran hydrogels (60/40 and 80/20) and cross-linked bovine tendon collagen and glycosaminoglycan scaffolds (Integra®; control scaffold) were analyzed. **Figure 3A** shows porosity. **Figure 3B** shows mechanics. **Figure 3C** shows representative H&E stained image on day 5 of low ratio (60/40) and high ratio (80/20). W = wound area, H = Hydrogel scaffold. Scale bars = 100 $\mu$m.

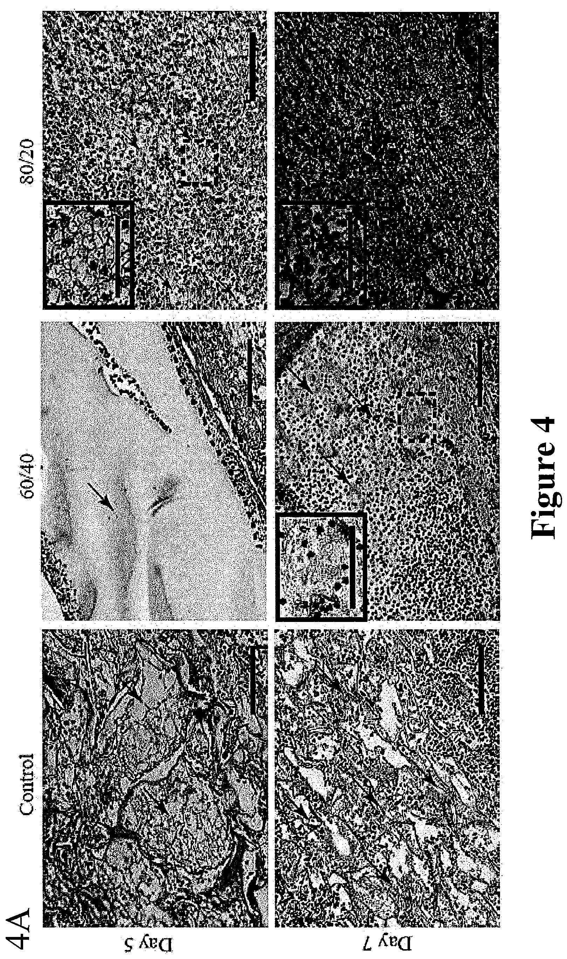

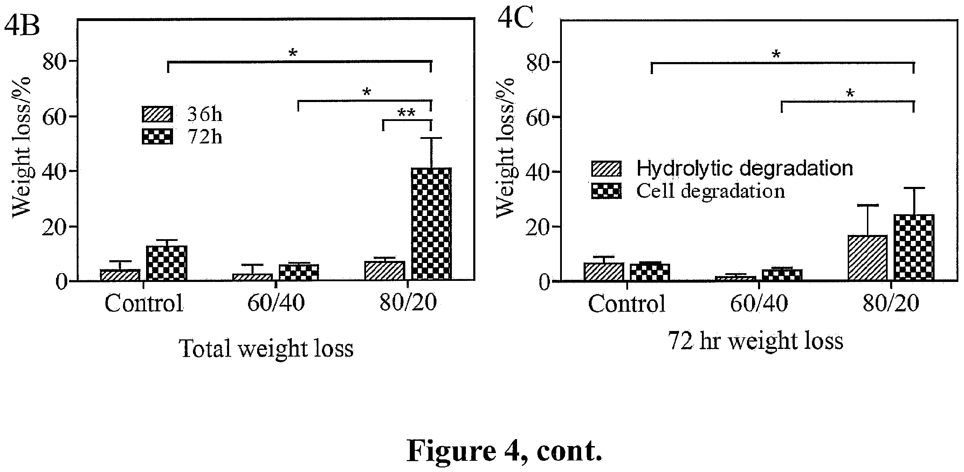

**Figure 4** shows hydrogel degradation. **Figure 4A** shows representative images of H&E stained histological sections of control scaffold (left), low ratio dextran hydrogel (middle) and high ratio dextran hydrogel (right) on days 5 and 7 of treatment show gel fragmentation (indicated by arrows and magnified inserts). *In vitro* degradation of Integra and hydrogels measured by the total change in scaffold mass **(Figure 4B)** the relative contribution of HL60 cells and hydrolysis after 72 hours **(Figure 4C).** Scale bars = 100 $\mu$m (40 $\mu$m in inserts).

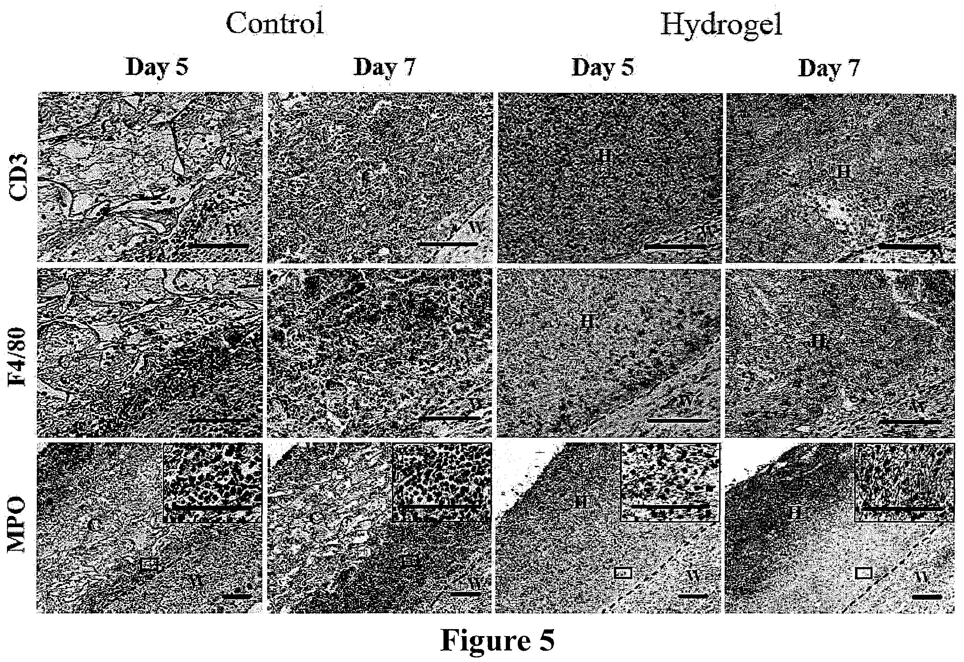

**Figure 5** shows inflammatory cell infiltration. Histological sections of control scaffold-treated and hydrogel-treated wounds (left and right, respectively) on days 5 and 7 of treatment, stained for CD3 (T cell), F4/80 (macrophage) and MPO (neutrophil). High magnification corresponds to boxed area in the low magnification images. The dotted line represents the interface between wound and dressing (control scaffold or hydrogel). W = Wound area, H= Hydrogel scaffold, and C = Control scaffold. Scale bars = 100 $\mu$m.

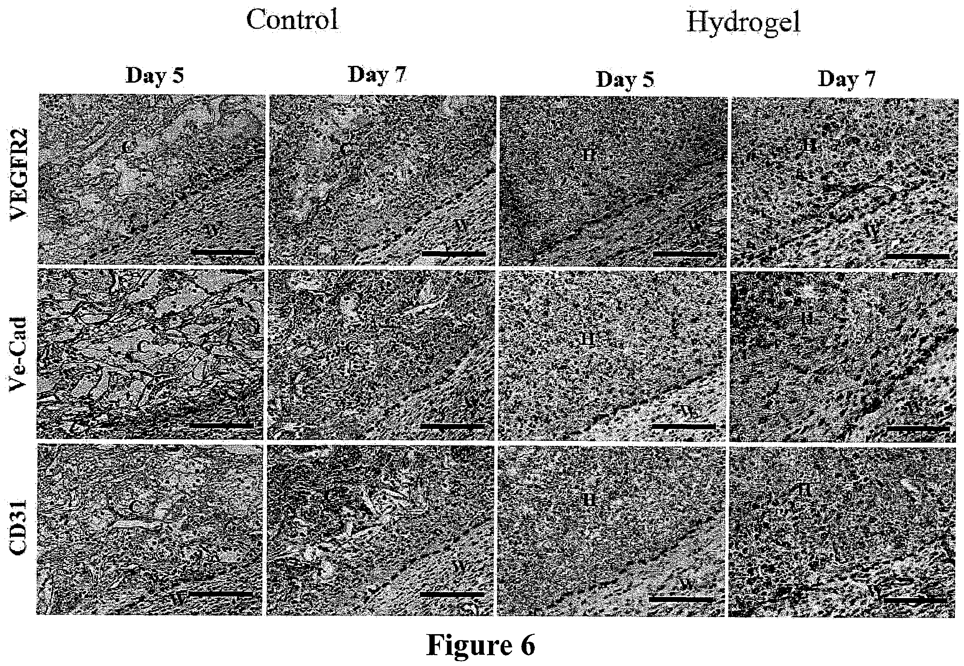

**Figure 6** shows angiogenic cell infiltration. Histological sections of control scaffold-treated and hydrogel-treated wounds (left and right, respectively) on days 5 and 7 of treatment, stained for VEGFR2 (upper panel), VE-Cad (middle panel), and CD31 (lower panel). The dotted line represents the interface between wound and control scaffold or hydrogel. W = wound area, H = Hydrogel scaffold, and C = Control scaffold. Scale bars = 100 $\mu$m.

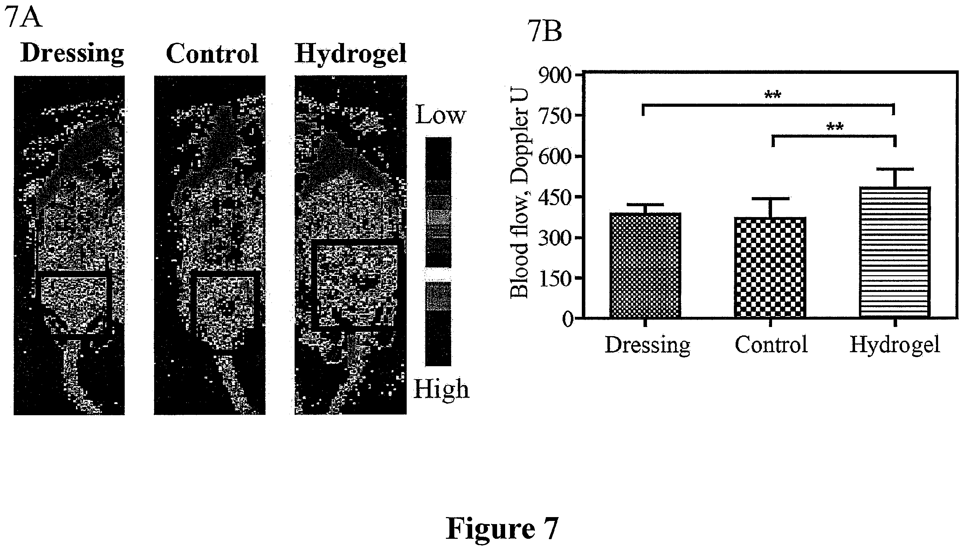

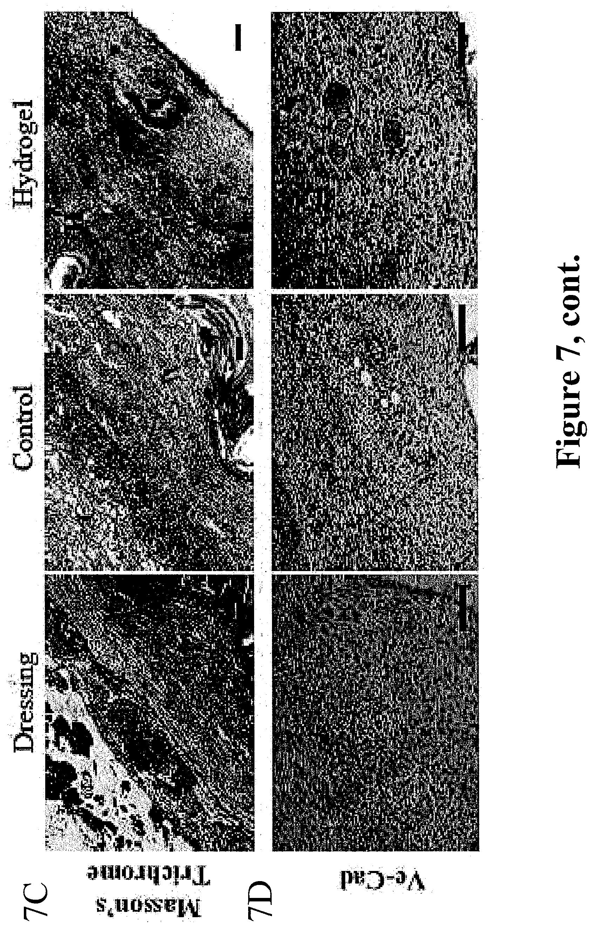

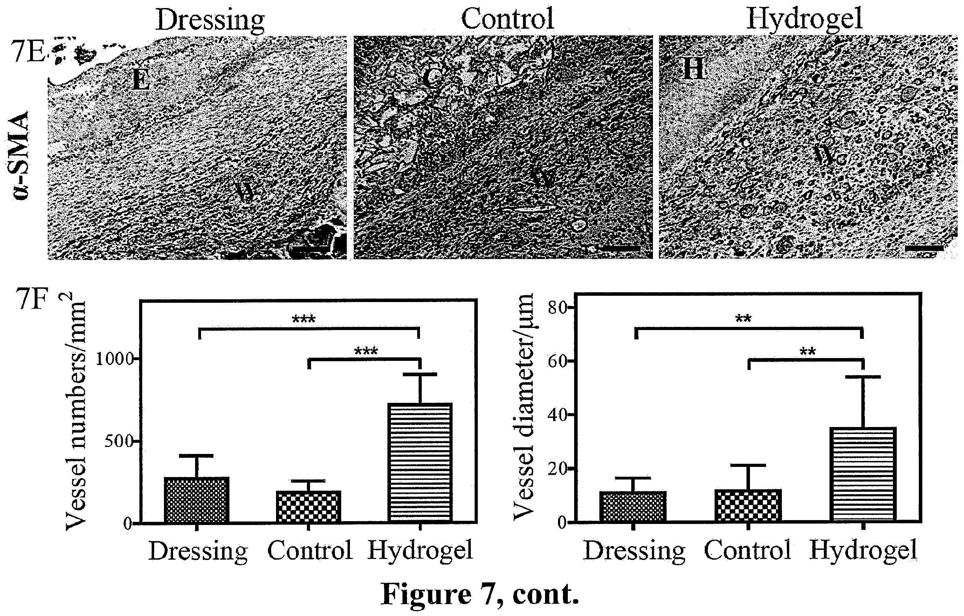

**Figure 7** shows angiogenic response in, on, or at day 7. **Figure 7A** and **7B** show Doppler images of angiogenic response to wound injuries **(Figure 7A),** and quantification **(Figure 7B).** The square indicates the wound area under Doppler. **Figures 7C** and **7D** show Masson's staining **(Figure 7C)** and VE-Cad staining **(Figure 7D)** of wound sites. Collagen layers were formed on the control (untreated) wounds, while no such layers formed on control scaffold-treated and hydrogel-treated wounds by day 7; functional blood cells in the hydrogel-treated wounds were observed. **Figure 7E** shows a photo of $\alpha$-SMA staining and **Figure 7F** shows quantification based on $\alpha$-SMA staining of the wound areas. W = Wound area, E-Eschar, H = Hydrogel scaffold, D= dressing, and C = Control scaffold. Significance levels were set at: *p < 0.05, **p < 0.01, and ***p < 0.001. Values shown are means ± SD. Scale bars = 100 $\mu$m.

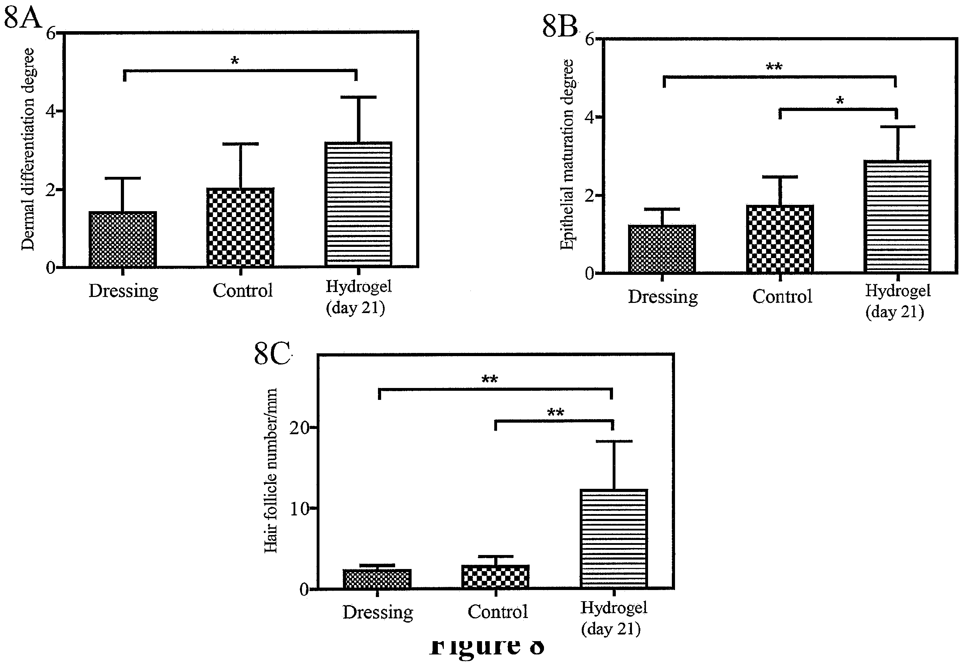

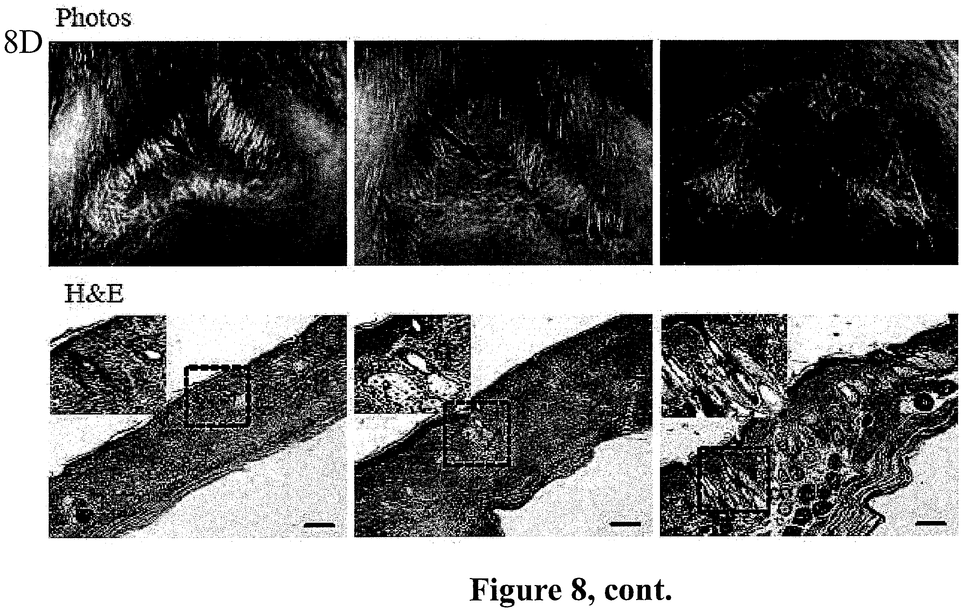

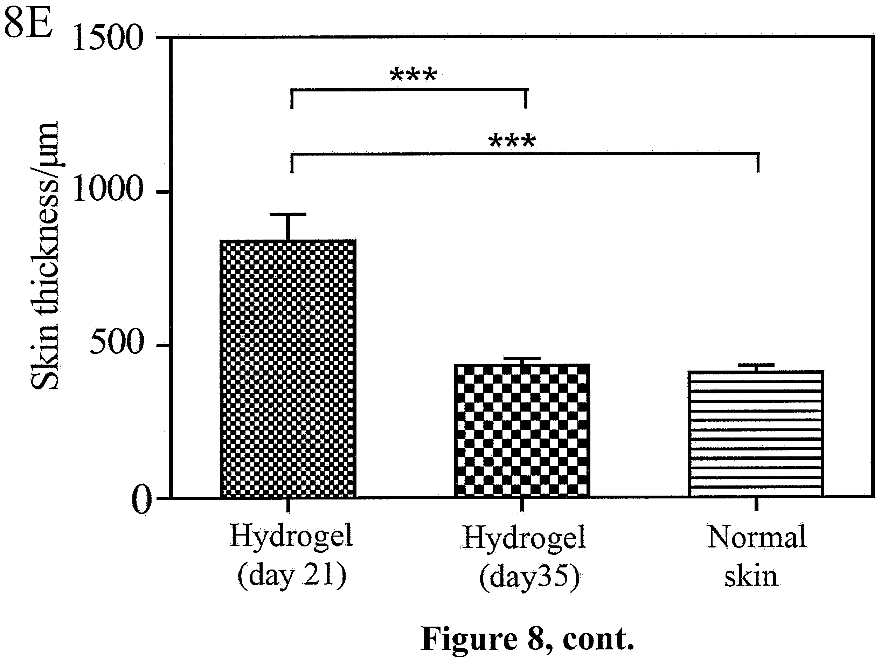

**Figure 8** shows an evaluation of regenerated skin structures. Quantification of skin structures in terms of dermal differentiation degree is shown in **Figure 8A,** and epithelial maturation degree **(Figure 8B),** and the number of hair follicles per milimeter **(Figure 8C); Figure 8D** shows a five-week-long study further demonstrating that dextran hydrogels promote complete skin regeneration with new hair growth, as shown by photos (arrows indicate the center of the original wound; upper panel) and H&E-stained histologic sections. High magnification corresponds to boxed area in the low magnification images. **Figure 8E** shows quantification of skin thickness after three-week and five-week-long treatment compared to normal mouse skin. Significance levels were set at: *p < 0.05, **p < 0.01, and ***p < 0.001. Values shown are means ± SD. Scale bars = 100 $\mu$m.

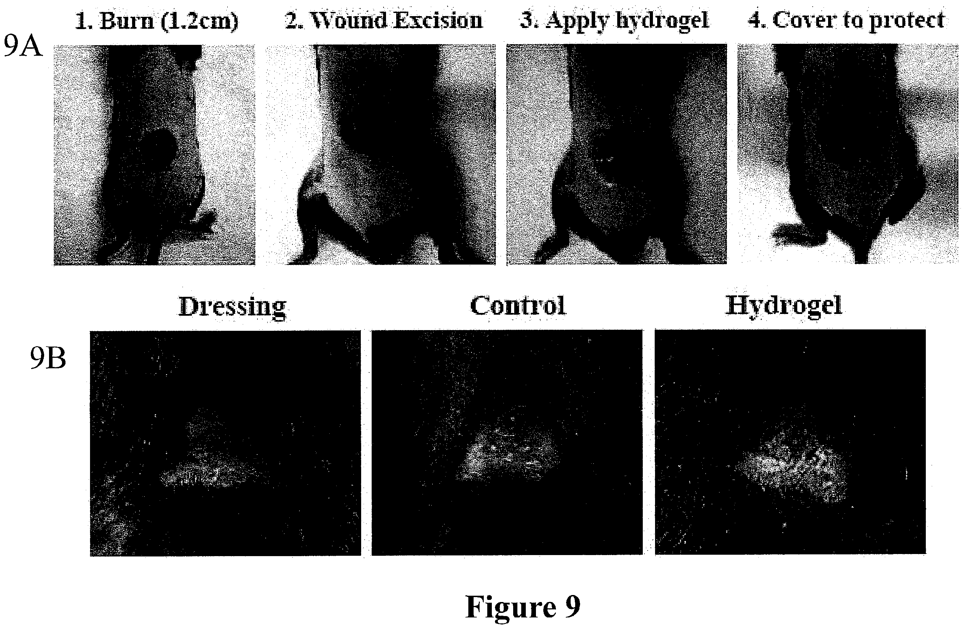

**Figure 9** shows Dextran hydrogel for burn wound healing. **Figure 9A** shows surgery procedure: wounds were placed on the posterior-dorsum of each mouse and performed burn wound excisions after 48 hours. Wounds were covered with either dextran hydrogels or control scaffold, followed by coverage with dressing. The control wounds were covered only with dressing. **Figure 9B** shows photo images of wound healing within 21 days demonstrate a more complete wound healing in burn wounds treated with dextran hydrogel than in wounds treated with control scaffolds or dressing alone.

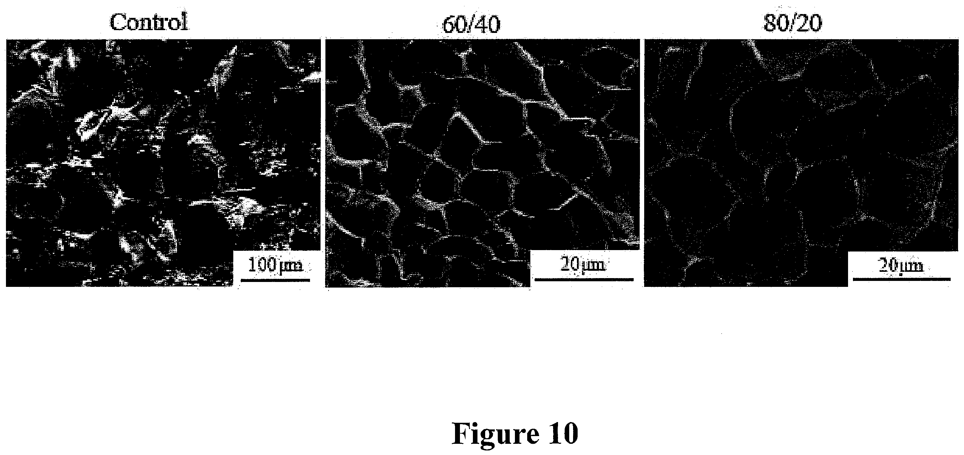

**Figure 10** shows scaffold porosity. Representative SEM images demonstrate scaffold porosity.

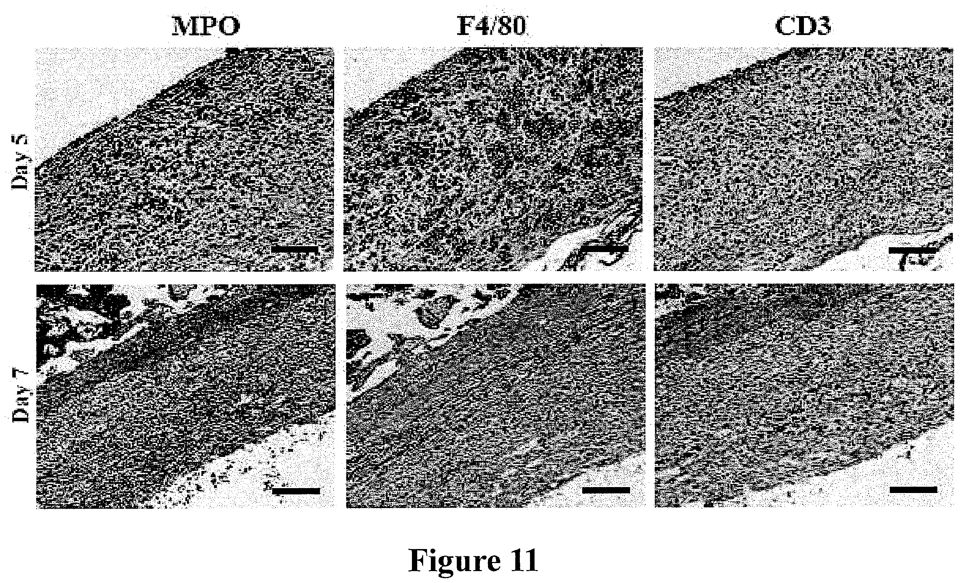

**Figure 11** shows inflammatory cell infiltration in control wounds covered with dressing. Neutrophils (MPO) and macrophages (F4/80) were clearly observed on day 5, while T cells (CD3) were observed on day 7. Scale bars = 100 $\mu$m.

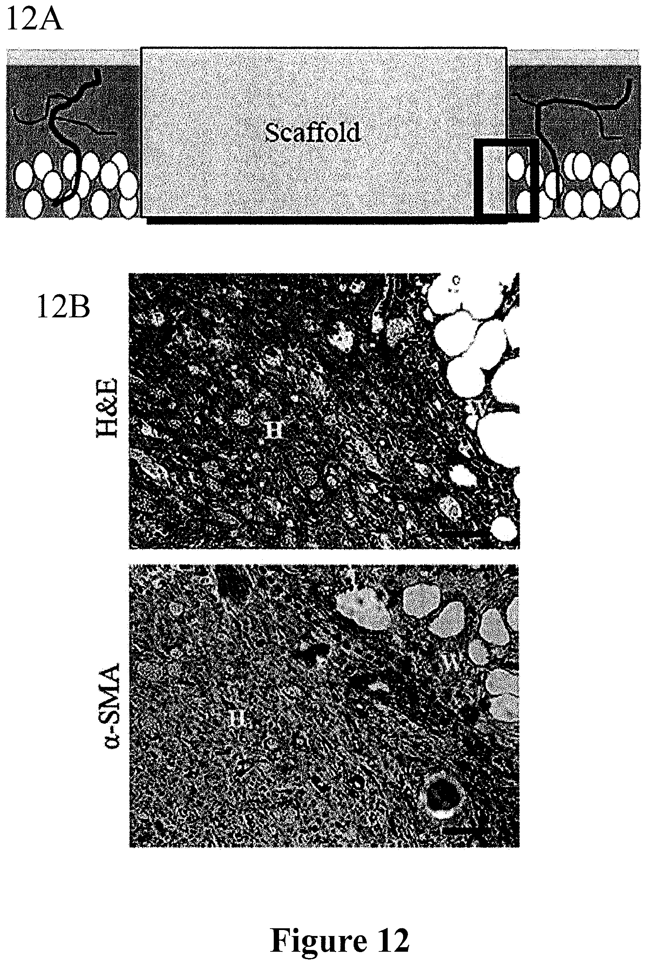

**Figure 12** shows illustration of angiogenic response at the interface between treatment and wounded skin. **Figure 12A** shows a schematic illustrating the location of the interface between the wound and hydrogel and **Figure 12B** shows H&E-stained histologic sections and $\alpha$-SMA staining showing the interface between the wound and hydrogel. The dotted line indicates the interfaces. W = Wound area, H = Hydrogel scaffold. Scale bars = 100 $\mu$m

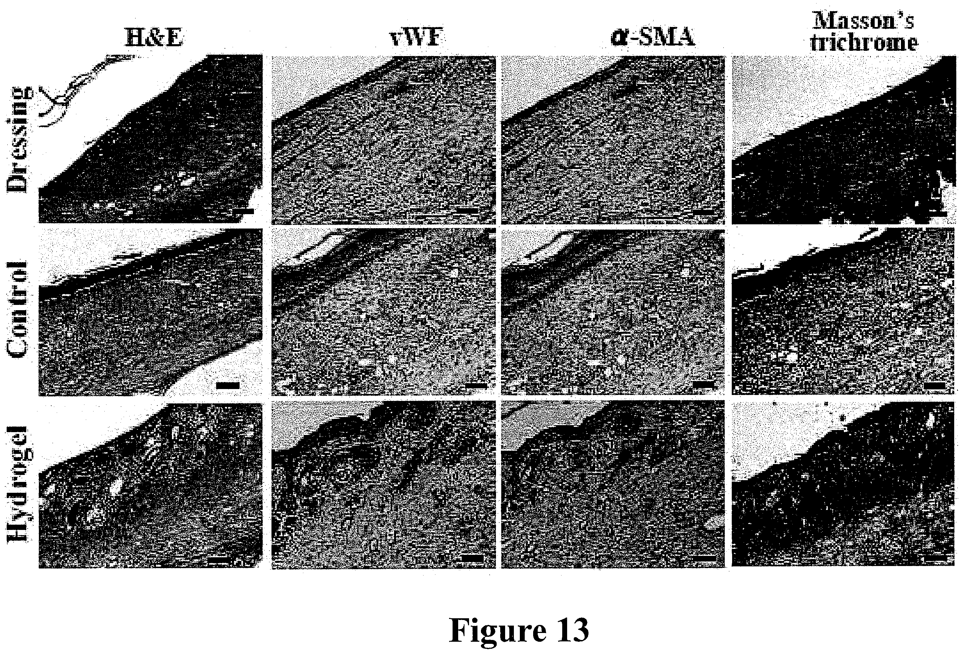

**Figure 13** shows skin regeneration within 21 days. Representative images of control dressing, control scaffold and hydrogel stained with H&E, vWF, $\alpha$-SMA, and Masson's trichrome. Scale bars = 100 $\mu$m.

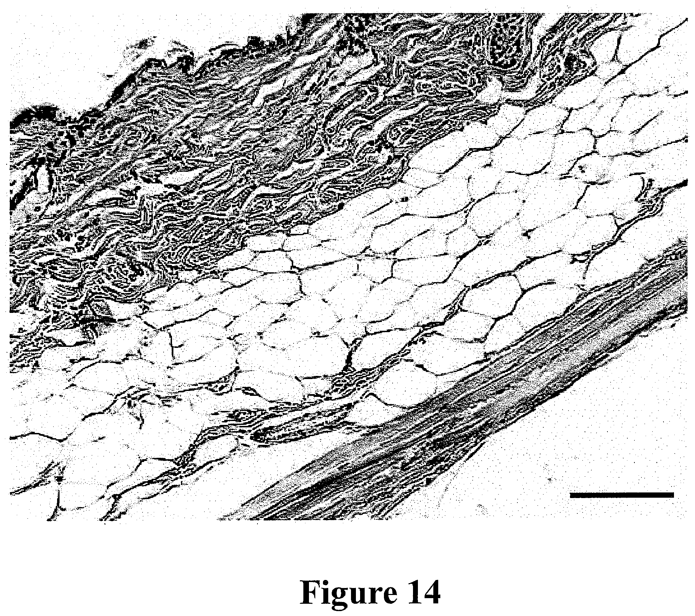

**Figure 14** shows normal mouse skin. H&E-stained histologic section of a 129S1/SvImJ mouse skin. Scale bar = 100 $\mu$m.

## DETAILED DESCRIPTION

Some embodiments of the current invention are discussed in detail below. In describing embodiments, specific terminology is employed for the sake of clarity. However, the invention is not intended to be limited to the specific terminology so selected. A person skilled in the relevant art will recognize that other equivalent components can be employed and other methods developed without departing from the broad concepts of the current invention. All references cited herein are incorporated by reference as if each had been individually incorporated. Headings used herein are provided for clarity and organizational purposes only, and are not intended to limit the scope of the invention.

*Definitions*

As used herein, the terms "prevent," "preventing," "prevention," "prophylactic treatment" and the like refer to reducing the probability of developing a disorder or condition in a subject, who does not have, but is at risk of or susceptible to developing a disorder or condition.

By "subject" is meant an animal. In some embodiments, a subject may be a mammal, including, but not limited to, a human or non-human mammal, such as a bovine, equine, canine, ovine, or feline.

By "therapeutic delivery device" is meant any device that provides for the release of a therapeutic agent.

As used herein, the terms "treat," treating," "treatment," "therapeutic" and the like refer to reducing or ameliorating a disorder and/or symptoms associated therewith. It will be appreciated that, although not precluded, treating a disorder or condition does not require that the disorder, condition or symptoms associated therewith be completely eliminated.

As used herein, the terms "promote," and "promoting" mean to increase the rate of, relative to a condition where no hydrogel is administered.

As used herein, the terms "reduce," and "reducing" when used in the context of a method of treatment mean decreasing the extent of or amount of, relative to a condition where no hydrogel is administered.

The term "$C_1$-$C_4$ alkyl" as used herein means straight-chain, branched, or cyclic $C_1$-$C_4$ hydrocarbons which are completely saturated and hybrids thereof such as (cycloalkyl)alkyl. Examples of $C_1$-$C_6$ alkyl substituents include methyl (Me), ethyl (Et), propyl (including *n*-propyl (n-Pr, $^n$Pr), *iso*-propyl (i-Pr, $^i$Pr), and cyclopropyl (c-Pr, $^c$Pr)), butyl (including *n*-butyl (n-Bu, $^n$Bu), *iso*-butyl (i-Bu, $^i$Bu), *sec*-butyl (s-Bu, $^s$Bu), *tert*-butyl (t-Bu, $^t$Bu), or cyclobutyl (c-Bu, $^c$Bu)), and so forth.

The term "3-6 membered ring" as used herein means a saturated, unsaturated, or aromatic ring having 3 to 6 atoms in the ring and at least two carbon atoms in the ring. Non-carbon atoms may include nitrogen, oxygen, sulfur, phosphorous and silicon. Some embodiments have 1 or 2 heteroatoms in the ring. In some embodiments, the 3-6 membered ring may be a "$C_3$ to $C_6$ ring" having 3-6 carbon atoms in the ring. Examples of 3-6 membered rings include cyclopropane, cyclopropene, epoxides, aziridine, tioepoxides, cyclobutane, cyclobutene, cyclobutadiene, oxetane, azetidine, thietane, cyclopentane, cyclopentene, cyclopentadiene, pyrrolidine, pyrroline, pyrrole, imidazole, tetrahydrofuran, dihydrofuran, furan, oxazole, oxadiazole, thiazole, thiadiazole, tetrahydrothiophene, dihydrothiophene, thiophene, cyclohexane, cyclohexene, cyclohexadiene, benezene, piperazine, pyridine, tetrahydropyran, pyran, thiane, thiopyran, morpholine, diazines (including pyridazines, pyrimidines, and pyrazines), and triazine rings. A polysaccharide having at least one substituted hydroxyl group can also be referred to as a "modified polysaccharide."

As used herein, "monomer," "saccharide monomer unit," "saccharide monomer," and the like are used to refer to a single saccharide unit of the polysaccharide. Saccharide monomers bearing a substituent are referred to herein as "modified monomers" or "modified saccharide monomers" or "modified saccharide monomer units."

## Therapeutic methods

Embodiments of the invention include methods of promoting skin regeneration by topically administering to a subject with an area of full thickness skin injury damaging the skin, a hydrogel on at least a portion of the injured area. The hydrogel used in the method is a crosslinked composition as defined in claim 1.

By topically administering the hydrogel to an injury damaging the skin, skin regeneration will be promoted. In other words, the rate of skin regeneration will be increased, when compared with healing without the hydrogel. The hydrogel can promote regeneration of both the dermis and epidermis of the skin.

Some embodiments of the invention include methods of promoting hair follicle regeneration by topically administering to a subject with an area of full thickness skin injury damaging the skin, a hydrogel on at least a portion of the injured area. The hydrogel used in the method is a crosslinked composition as defined in claim 2.

By topically administering the hydrogel to an injury damaging the skin, hair follicle regeneration will be promoted. In other words, more hair follicles will be regenerated using the hydrogel, when compared with healing without the hydrogel.

Other embodiments of the invention include methods of reducing scarring by topically administering to a subject with an area of full thickness skin injury damaging the skin, a hydrogel on at least a portion of the injured area. The hydrogel used in the method is a crosslinked composition as defined in claim 3.

By topically administering the hydrogel to an injury damaging the skin, scarring will be reduced when compared with healing without hydrogel treatment.

An injury damaging the skin includes any injury where the skin has been damaged, removed, physically destroyed, functionally destroyed, or where the continuity of the skin has been interrupted, and is also referred to herein as a wound. In some aspects, the injury may extend below the deeper layer of the skin to muscle, tendon, or bone, and scarring is likely to result when healed. These are examples of full thickness skin injuries. Injuries also include various types of open wounds, including skin avulsions, lacerations, abrasions, punctures, or incisions and various types of burns caused by various factors, such as, for example, thermal, electrical, chemical, or radiation, and including second degree burns and third degree burns. In the present invention, the injury is a full thickness skin injury. In these and other exemplary embodiments of the invention, the injury is a second-degree burn or a third-degree burn.

The hydrogel may be administered to at least a portion of the injured area. In some embodiments, a pre-formed hydrogel is placed on the injured area. The hydrogel or crosslinked composition may be formed in a particular shape, for example as ovoid, sphere, disc, sheet or other structure. In some embodiments, the hydrogel or crosslinked composition is shaped to cover a portion of the injured area or the entire injured area. In embodiments, the hydrogel or crosslinked composition is placed on the injured area such that an edge of the hydrogel or crosslinked composition is in contact with an uninjured area immediately adjacent to the injured area. In other embodiments, the hydrogel or crosslinked composition is placed on the injured area such that an edge of the hydrogel or crosslinked composition overlaps, i.e. extends over, an uninjured area immediately adjacent to the injured area.

In some embodiments, the hydrogel may be administered to extend the hydrogel over an uninjured area, in addition to the injured area. In some embodiments, the entire injured area may be covered by the hydrogel, with or without extension over an uninjured area.

In some embodiments, an un-crosslinked composition may be applied to the injured area and crosslinked in place. In some embodiments, the composition may be administered to a subject as an uncrosslinked composition, followed by crosslinking. In this way, the hydrogels may be molded to a particular shape, based on the location of administration. In some embodiments, the composition is crosslinked prior to administration.

The hydrogel may be kept in place with a dressing. The dressing may protect the wound from bacterial infection, control evaporative water loss and prevent dehydration, control the permeability of oxygen and carbon dioxide and absorb wound exudate.

In some embodiments, the wound may be excised prior to administration of the hydrogel.

Any hydrogels described further herein may be used in any of the above methods.

Hydrogels and hydrogel forming compositions

Hydrogels may be formed from any hydrogel forming composition described herein by crosslinking the composition, as described further.

The hydrogel may be crosslinked between polysaccharide molecules, or between polysaccharide molecules and one or more other crosslinkable molecules. Other embodiments include compositions of a crosslinked blend of polysaccharide and a second crosslinkable molecule.

In some embodiments, the second crosslinkable molecule is a polymer. As used herein, a "crosslinkable" molecule or polymer is a material bearing at least two reactive groups capable of forming a covalent bond or crosslink with the crosslinkable moiety of the polysaccharide. Examples of crosslinkable molecules include, for example, acrylate groups and, methacrylate groups. Polymers having at least two crosslinkable groups are useable, such as, poly(alkyleneglycol) diacrylate, poly(alkyleneglycol) dimethacrylate. Specific examples include poly(ethylene glycol) diacrylate. Other polymers, both degradable and nondegradable may be used. Examples include hyaluronic acid, chitosan or poly(ester amide) polymers having crosslinkable moieties. Crosslinkable moieties other than double bonds may also be used, such as thiol containing polymers. Thiol containing polymers may crosslink with double bond crosslinking moieties on the polysaccharide, or thiol-containing moieties on the polysaccharide. This chemistry may be useful for non-photocrosslinking where UV irradiation is not desirable.

When a second crosslinkable molecule is used, there is a non-saccharide linking moiety between the crosslinked polysaccharides. For example, when the second crosslinkable molecule is poly(ethylene glycol) diacrylate, the linking moiety is a polyethyelene glycol. In some embodiments the crosslinked composition is a hydrogel. In other embodiments, the crosslinked composition is a hydrogel comprising a blend of polysaccharide and poly(ethylene glycol) diacrylate.

In some embodiments $R^7$ is H. In some embodiments, the second crosslinkable molecule is poly(ethylene glycol) diacrylate. In other embodiments, $R^7$ is H, and the second crosslinkable molecule is poly(ethylene glycol) diacrylate.

A "hydrogel forming composition" as used herein means a composition capable of forming a solid hydrogel when crosslinked, rather than a fluid-like gel. Persons skilled in the art will generally be able to distinguish a solid hydrogel from a fluid-like hydrogel. For instance, a "solid hydrogel" is capable of maintaining its shape after crosslinking, or has sufficient structure that mechanical properties, such as the modulus may be measured. However, by way of example, and not limitation, a solid hydrogel may be considered a hydrogel having an increase in mechanical strength. Alternatively, a solid hydrogel may be a gel with a modulus greater than about 200 Pa, greater than about 500 Pa, greater than about 700 Pa, or greater than about 1000 Pa. In some embodiments, the degree of substitution of formula (III) is about 0.2 or less, as described below.

Hydrogels can be formed by crosslinking through use of, for example, chemical and photochemical means. Photochemical crosslinking can offer some advantages including reduction in the exposure to chemical initiators or other reagents, and greater control over degree of crosslinking by having direct control over exposure to light. In many cases, it is still advantageous to reduce the exposure time to UV radiation. For this reason, certain embodiments include hydrogels and hydrogel forming compositions that form solid hydrogels in a particular period of time. For instance, the compositions may form solid hydrogels in less than about 1 hour, less than about 45 minutes, less than about 30 minutes, or less than about 20 minutes using photoirradiation at 365 nm with a lamp power of about 100W.

Other embodiments include a hydrogel forming composition having a second substituted hydroxyl group having the formula (IV), where formula (III) and formula (IV) are different, and the substituted hydroxyl group of formula (III) and formula (IV) may be on the same or different monomers. Formula (IV) has the structure

$$Y\text{-}(CR^2R^3)_n\text{-}Z \qquad \text{(IV)}$$

where Y is $-O_1-$ or $-O_1C(O)-$, or $-O_1C(O)NR^1-$, $O_1$ is the oxygen atom of said substituted hydroxyl group, and $R^1$ is hydrogen or $C_1$-$C_4$ alkyl; n = 1, 2, 3, or 4; Z is selected from the group consisting of $-CO_2H$ or $NR^4R^5$, where $R^4$ and $R^5$ are independently hydrogen or $C_1$-$C_4$ alkyl. $R^2$ and $R^3$ are independently hydrogen, $C_1$-$C_4$ alkyl, or may combine to form a 3-6 membered ring, and when n>1, $R^2$ and $R^3$ on adjacent carbons may form a double or triple bond, or $R^2$ and $R^3$ on different carbon atoms may form a 3-6 membered ring. In some embodiments, Z is $NR^4R^5$. In other embodiments, formula (IV) is $-O_1-(CH_2CH_2)-NH_2$.

Other embodiments include a hydrogel having at least about 80% of at least one polysaccharide portion and up to about 20% poly(ethylene glycol) diacrylate portions, where the polysaccharide portion is derived from a polysaccharide with at least one monomer having at least one substituted hydroxyl group, and the substituted hydroxyl group has the formula (III). The hydrogel is formed by photocrosslinking. As discussed above, formula (III) has the structure $-O_1-C(O)NR^7-CH_2CH=CH_2$ where $O_1$ is the oxygen atom of said substituted hydroxyl group and $R^7$ is hydrogen or $C_1$-$C_4$ alkyl.

Other embodiments include a hydrogel having at least about 80% of at least one polysaccharide portion and up to about 20% poly(ethylene glycol) diacrylate portions, as discussed above, where the polysaccharide has a second substituted hydroxyl group having the formula (IV), where formula (III) and formula (IV) are different, and the substituted hydroxyl group of formula (III) and formula (IV) may be on the same or different monomers. As discussed above, formula (IV) has the structure $Y\text{-}(CR^2R^3)_n\text{-}Z$ where Y is $-O_1-$ or $-O_1C(O)-$, or $-O_1C(O)NR^1-$, $O_1$ is the oxygen atom of said substituted hydroxyl group, and $R^1$ is hydrogen or $C_1$-$C_4$ alkyl; n = 1, 2, 3, or 4; Z is selected from the group consisting of $-CO_2H$ or $NR^4R^5$, where $R^4$ and $R^5$ are independently hydrogen or $C_1$-$C_4$ alkyl. $R^2$ and $R^3$ are independently hydrogen, $C_1$-$C_4$ alkyl, or may combine to form a 3-6 membered ring, and when n>1, $R^2$ and $R^3$ on adjacent carbons may form a double or triple bond, or $R^2$ and $R^3$ on different carbon atoms may form a 3-6 membered ring. In some embodiments, Z is $NR^4R^5$. In other embodiments, formula (IV) is $-O_1-(CH_2CH_2)-NH_2$.

In general, biocompatible hydrogels having a higher composition of polysaccharide are advantageous, because a greater portion of the hydrogel can be metabolically degraded. This higher composition also results in greater control of the amount of any added components released from the hydrogel matrix, because more of the matrix can be metabolized *in vivo*.

In some embodiments, the hydrogel forming composition may produce a hydrogel with a swelling ratio of greater than about 1200%. The swelling ratio may be determined gravimetrically by immersing a dry hydrogel sample of known weight in distilled water, and measuring the increase in weight until the weight no longer changes. The swelling ratio can then be calculated according to formula (1)

$$\text{Swelling ratio} = ((W_{s,t} - W_d)/W_d) \times 100\% \qquad (1)$$

where $W_d$ is the weight of dry hydrogels, and $W_{s,t}$ is the weight of swollen hydrogels at time t. The hydrogels were assumed to reach a state of swelling equilibrium when there was no difference in swelling ratio between two adjacent intervals.

In some embodiments, the composition may produce a hydrogel having a swelling ratio of greater than about 1500%, greater than about 1700% or greater than about 1900%. The hydrogels of the present invention may have a swelling ratio of greater than about 1200%, greater than about 1500, greater than about 1700%, or greater than about 1900%. In general, an increased swelling ratio results in an increased release rate of any added components such as proteins.

In some embodiments, the at least one hydroxyl-substituted saccharide monomer is a glucopyranose monomer. The glucopyranose monomer may be substituted at any available free hydroxyl group, or may be substituted on more than one available free hydroxyl group. The glucopyranose monomer may be incorporated into the polysaccharide in any suitable orientation, for example, via a 1,2-, 1,3-, 1,4-, 1,6-, or other linkage.

The polysaccharide is dextran. In some embodiments, the dextran has an average molecular weight of at least about 20,000. The dextran may have an average molecular weight of at least about 30,000, at least about 40,000, at least about 50,000, or at least about 60,000. The dextran may have an average molecular weight less than about 200,000, less than about 150,000, or less than about 100,000. The dextran may have a molecular weight between any two endpoints. The molecular weight may be number average or weight average. For instance, the dextran molecule may have an average molecular weight between about 20,000 and about 200,000, between about 20,000 and about 100,000 or between about 40,000 and about 70,000.

In some embodiments, the composition further comprises poly(ethylene glycol) diacrylate. In other embodiments, the poly(ethylene glycol) diacrylate has a molecular weight of at least about 2000, at least about 4000, at least about 6000, at least about 8000, or at least about 10,000. In some embodiments, the poly(ethylene glycol) diacrylate has a molecular weight less than about 50,000, less than about 20,000, or less than about 15,000. The poly(ethylene glycol) diacrylate may have an a molecular weight of between any two previously disclosed endpoints. The molecular weight may be number average or weight average. In general, larger poly(ethylene glycol) polymers are cleared more slowly from the body by the kidneys. Larger poly(ethylene glycol) may result in hydrogels with a looser structure, larger pore size, and higher swelling. Persons skilled in the art can use routine experimentation to determine and select a poly(ethylene glycol) or poly(ethylene glycol) diacrylate to provide desired physical properties for a hydrogel according to the invention.

Polysaccharides with low degree of substitution

In some embodiments, the hydrogel is formed of a polysaccharide with low degree of substitution of the substituent of formula (III) as described further.

Hydrogels used in the invention include a polysaccharide with at least one monomer having at least one substituted hydroxyl group, wherein the substituted hydroxyl group has the formula (III), and wherein the degree of substitution of formula (III) on the polysaccharide is less than about 0.2; wherein formula (III) is $-O_1-C(O)NR^7-CH_2CH=CH_2$ and $O_1$ is the oxygen atom of said substituted hydroxyl group and $R^7$ is hydrogen or $C_1-C_4$ alkyl. "Degree of substitution" (DS) is defined as the average number of substituted hydroxyl groups per saccharide monomer. A degree of substitution less than about 0.2 means that the number of substituted hydroxyl groups having the structure of formula (III) in the polysaccharide, divided by the total number of monomers in the polysaccharide is less than about 0.2. The degree of substitution can be calculated from the NMR spectrum. For example, the ratio of the sum of the normalized, integrated intensities of the hydroxyl group peaks to the normalized, integrated intensities of the anomeric proton peak is subtracted from the number of unsubstituted hydroxyl groups in an unmodified monomer unit to determine the degree of substitution. For dextran polysaccharides, for example, each dextran monomer unit has three hydroxyl groups. If, for example, the sum of the integrated intensities of the hydroxyl peaks was 11, and the integrated intensity of the anomeric proton was 4, the ratio would be 2.75. This value (2.75) is subtracted from the total number of hydroxyls (3), to calculate the degree of substitution (3-2.75 = 0.25). This also corresponds to an average of one substituted hydroxyl group for every 4 monomer units. In some embodiments, the degree of substitution may be between about 0.01 and about 0.2. In other embodiments, the degree of substitution is less than about 0.18, less than about 0.15, less than about 0.13, or less than about 0.10. In some embodiments, the degree of substitution is greater than about 0.01, greater than about 0.03, greater than about 0.05, or greater than about 0.07. Embodiments of the invention may have any combination of maximum and minimum previously specified.

In some embodiments, $R^7$ is hydrogen.

In some embodiments, the polysaccharide further includes a second substituted hydroxyl group having the formula (IV), where formula (III) and formula (IV) are different, and the substituted hydroxyl group of formula (III) and formula (IV) may be on the same or different monomers. Formula (IV) is $Y-(CR^2R^3)_n-Z$, where Y is $-O_1-$ or $-O_1C(O)-$, or $-O_1C(O)NR^1-$, $O_1$ is the oxygen atom of said substituted hydroxyl group, and $R^1$ is hydrogen or $C_1-C_4$ alkyl; n = 1, 2, 3, or 4; Z is selected from the group consisting of -CO$_2$H or NR$^4$R$^5$, where R$^4$ and R$^5$ are independently hydrogen or C$_1$-C$_4$ alkyl.

In some embodiments, Z is NR$^4$R$^5$. In some embodiments, formula (IV) is -O$_1$-(CH$_2$CH$_2$)-NH$_2$.

It is advantageous to prepare hydrogels that utilize high percentages (e.g., greater than 80%) of polysaccharides. For example, such hydrogels exhibit improved biocompatibility and biodegradation. However, conventional polysaccharides, when used to with crosslinking agents, often do not have favorable gel forming characteristics. Polysaccharides with low degrees of substitution of a crosslinking moiety on a hydroxyl group have been found to form hydrogels with high polysaccharide content. No particular minimum degree of substitution is required, so long as a solid gel can be formed.

## Added components

In exemplary embodiments of methods according to the invention, a hydrogel or crosslinked composition is used as described above without any additional components.

In other embodiments, the hydrogels discussed previously further include one or more of a protein, oligonucleotide, or pharmaceutical agent. In some embodiments, the crosslinked composition comprises a protein, oligonucleotide, or pharmaceutical agent that is released from the composition over time, when present in an environment, for example an aqueous environment, having a lower concentration of the protein, oligonucleotide, or pharmaceutical agent. "Released from the composition" as used herein, means that the concentration of protein oligonucleotide, or pharmaceutical agent in the crosslinked composition decreases. The aqueous environment may be, for instance, a buffer, such as phosphate buffered saline (PBS) or other buffer. The buffered solution may also include dextranase enzyme or dextranase enzyme may be added. The "aqueous environment" also includes situations where the crosslinked composition is administered to a subject for the purpose of delivering a protein, oligonucleotide, or pharmaceutical agent to the subject. The environment into which the protein, oligonucleotide, or pharmaceutical agent is released can be blood, lymph, tissue, for example an organ tissue, gastric juices, or other environment.

In some embodiments, when a crosslinked composition of modified polysaccharide, poly(ethylene glycol) diacrylate and protein is incubated at 37 °C in phosphate buffered saline (PBS), less than 10% of the protein (by weight) is released from the crosslinked composition in the first 24 hours.

In some embodiments, the hydrogel further comprises a protein, oligonucleotide or pharmaceutical agent. In general, any protein, oligonucleotide or pharmaceutical agent which may be delivered by a hydrogel may be delivered by the compositions of the present invention.

In some embodiments, the hydrogel further comprises a protein. Examples of proteins that may be delivered by hydrogels include bovine serum albumin (BSA) or ovalbumin. In some embodiments, the protein is a therapeutic protein, such as insulin or immunoglobulins (such as IgG). In some embodiments, the therapeutic protein is a growth factor. Examples of growth factors include vascular endothelial growth factor (VEGF), insulin growth factor (IGF), keratinocyte growth factor (KGF), stromal-cell derived factor (SDF), and angiopoetin (Ang). In some embodiments, the oligonucleotide is an antisense oligonucleotide.

In some embodiments, the hydrogel further comprises a pharmaceutical agent. In some embodiments, the pharmaceutical agent is an antibiotic. When the hydrogel includes an antibiotic, the antibiotic may be, for example, an antibacterial, antifungal, antiviral, or antimicrobial agent to prevent or reduce infection of the wound.

## Preparation

The polysaccharides described above may be prepared according to methods known in the art. For instance, the unsubstituted polysaccharide bearing a reactive hydroxyl group may be reacted with a reagent bearing a crosslinkable moiety to produce the structure of formula (III). The reagent may react with the free hydroxyl group directly, or the reagent or hydroxyl group may be activated to react with the reagent.

Polysaccharide having substituted hydroxyl groups with the structure of formula (III) may be prepared, for example, by reacting a polysaccharide with allylisocyanate in the presence of an activator, such as dibutyltin dilaurate (DBTDL). The degree of substitution is controlled by reducing the mole ratio of allylisocyanate to polysaccharide to produce the desired degree of substitution.

The modified polysaccharide having the substituent of formula (III) may then be reacted with a reagent to form a substituent of formula (IV) using reagents discussed previously. Substituents of formula (IV) where Z is NR$^5$R$^6$ may be prepared, for example, by reacting the modified polysaccharide with an amine bearing reagent similar to those described previously having a carboxylic acid. For example, the polysaccharide may be reacted with 2-bromoethylamine hydrobromide to form the substituent having the formula -O$_1$-(CH$_2$CH$_2$)-NH$_2$. Alternatively, a polysaccharide may be reacted with a reagent to form a substituent of formula (IV), and then reacted with a reagent to form a hydroxyl group with the structure of formula (III).

The polysaccharide may be purified, for example, by precipitation, or by chromatography, such as size exclusion chromatography.

Crosslinked compositions may be prepared by crosslinking the modified polysaccharide using any suitable chemistry, based on the crosslinking moiety. In some embodiments, where the crosslinking moiety comprises a double bond, photocrosslinking may be used to crosslink the composition. The composition may further include a second crosslinkable molecule or polymer. The second crosslinkable molecule or polymer should have at least two crosslinkable groups capable of forming crosslinks with the crosslinkable moieties of the modified polysaccharide.

Proteins, oligonucleotides or pharmaceutical agents may be incorporated into the crosslinked composition. In some cases, the protein, oligonucleotide or pharmaceutical agent are incorporated by soaking the crosslinked compositions in a solution containing the protein, oligonucleotide or pharmaceutical agent. In other cases, the protein, oligonucleotide or pharmaceutical agent may be present in a solution containing uncrosslinked modified polysaccharide, with or without a second crosslinkable molecule. The composition is then crosslinked, for example, by photocrosslinking, to form a crosslinked composition including the protein, oligonucleotide or pharmaceutical agent.

In exemplary embodiments, the modified polysaccharide is a modified dextran molecule, and the second crosslinkable molecule is based on poly(ethylene glycol), for example poly(ethylene glycol) diacrylate (PEGDA).

The preparation of dextran-based hydrogels is illustrated using Dex-AI/PEGDA hydrogels, as shown below in **Figure 1.** The objective of this step was to prepare the dextran-based hydrogels through the photocrosslinking of dextran-based precursors and PEGDA, using a long-wave (365 nm) UV lamp. A synthetic polymer precursor was introduced to have both synthetic and natural polymers occur in a single resulting hydrogel, thus obtaining tunable properties. Among synthetic polymer precursors, PEG has been extensively employed for many biomedical applications, due to its unique amphiphilic, biocompatible, but nonbiodegradable properties. Though PEG is not biodegradable, it can be readily excreted from the body via kidney and liver, thereby making it more suitable for biomedical applications. In addition, PEG has been employed to improve biocompatibility Zhang et al., Biomaterials, 2002, vol. 23, p. 2641-2648), to increase bioactivity (Muslim et al., Carbohydr. Polym., 2001, vol. 46. p. 323-330), and to reduce immunogenicity (Hu et al., Int. J. Biochem. Cell. Biol., 2002, vol. 34, p. 396-402).

Including a synthetic polymer such as poly(ethylene glycol) in the crosslinked composition provides the capability to tune the properties of the resulting hydrogel. Tunable properties include mechanical properties, such as the swelling and modulus of the hydrogel. Other properties influenced by the type of synthetic polymer include crosslinking density and the release profile of any incorporated protein, oligonucleotide or pharmaceutical agent.

Properties of the crosslinked composition may be varied by varying the components of the composition, using a different modified polysaccharide, or changing the degree of substitution of one or more substituents on the modified polysaccharide. Other properties may be adjusted by varying the size of the polysaccharide, or the size of the second crosslinkable compound or polymer.

From the foregoing description, it will be apparent that variations and modifications may be made to the invention described herein to adopt it to various usages and conditions. Such embodiments are also within the scope of the following claims.

The recitation of a listing of elements in any definition of a variable herein includes definitions of that variable as any single element or combination (or subcombination) of listed elements. The recitation of an embodiment herein includes that embodiment as any single embodiment or in combination with any other embodiments or portions thereof.

Terms listed in single tense also include multiple unless the context indicates otherwise.

The examples disclosed below are provided to illustrate the invention but not to limit its scope. Other variants of the invention will be readily apparent to one of ordinary skill in the art and are encompassed by the appended claims. All publications, databases, and patents cited herein are hereby incorporated by reference for all purposes.

Methods for preparing, characterizing and using the compounds of this invention are illustrated in the following Examples. Starting materials are made according to procedures known in the art or as illustrated herein. The following examples are provided so that the invention might be more fully understood. These examples are illustrative only and should not be construed as limiting the invention in any way.

## EXAMPLES

*Materials*

Dextran (MW 70,000) and allyl isocyanate (AI) were purchased from Sigma Chemical Co. (St. Louis, MO). Dextran was dried the an oven for 30 minutes at 60 °C before reaction. Dimethyl sulfoxide (DMSO), dibutyltin dilaurate (DBTDL), 2-bromoethylamine hydrobromide (BEAHB), triethylamine, acryloyl chloride, polyethylene glycol (PEG; MW 4,000), and other chemicals were purchased from Aldrich Chemical Co. (Milwaukee, WI) and used as received. The photoinitiator 2-hydroxy-1-[4-(hydroxyethoxy)phenyl]-2-methyl-1-propanone was obtained from Ciba Specialty Chemicals Corp. (Tarrytown, NY). Male eight-week-old 129S1/SvImJ mice were obtained from The Jackson Laboratory (Bar Harbor, ME). Integra wound dressing was purchased from Integra Life Sciences Co. (Plainsboro, NJ) and DuoDerm ultra thin dressing from ConvaTec Co. (Skillman, NJ).

*Statistics*

All measurements were obtained from at least six different slides or mice, with multiple readings for each data point (as detailed throughout the manuscript). The number of animals (n) refers to the number per group. Blood flow was quantified using Doppler, the number and diameter of angiogenic blood vessels, the degrees of dermal differentiation, epithelial maturation, and the number of regenerated hair follicles. Either one-way ANOVA were performed with Tukey's post tests or two-way ANOVA with Bonferroni post tests where appropriate (GraphPad Prism 4.02). Significance levels, determined using post tests between controls, hydrogels, and Integra, were set at: $*p<0.05$, $**p<0.01$, and $***p<0.001$. All graphical data is reported.

EXAMPLE 1

**Preparation of Dex-AE/PEGDA hydrogel**

Dex-AE/PEGDA hydrogels were prepared as previously reported (Sun et al., Journal of Biomedical Materials Research Part A, vol. 93A, no. 3, pp. 1080-1090, 2010; Sun et al., Biomaterials, vol. 32, no. 1, pp. 95-106, 2011). Dex-AE/PEGDA was dissolved at the ratio of 60/40 and 80/20 into phosphate buffered saline (PBS) containing 0.1 percent (w/w) 2-methyl-1-[4-(hydroxyethoxy)phenyl]-2-methyl-1-propanone (Irgacure 2959, Ciba). The mixture was pipetted into a sterile mold (70 $\mu$L volume per well) to obtain discs measuring 8 mm in diameter by 2 mm thick and photopolymerized (approximately 10 mW/cm$^2$ of UV light for ten minutes; Black-Ray, UVP, Upland, CA). The resulting hydrogels were removed from the mold and immersed in sterile PBS solution before application onto wounds.

**Mechanical study of scaffolds**

The mechanical properties of the scaffold samples (n=3) were determined as previously established (Sun et al., Journal of Biomedical Materials Research Part A, vol. 93A, no. 3, pp. 1080-1090, 2010; Sun et al., Biomaterials, vol. 32, no. 1, pp. 95-106, 2011), using a Q800 Dynamic Mechanical Analyzer (TA Instruments, New Castle, DE) in unconfined submersion compression mode. Briefly, the diameter of each swollen hydrogel disk was determined using a digital caliper, and the sample was immersed in a PBS bath between unconfined parallel compression platens. Scaffold samples were compressed at a rate of 10% of thickness/minute until they reached 80 of their initial thickness. The modulus was then calculated as the ratio of the stress-strain curve at the linear portion of the curve.

**Scanning electron microscope (SEM) and pore size determination**

The ultrastructure of the scaffold was studied using SEM (FEI Quanta ESEM 200). The hydrogels were swelled in phosphate-buffered saline (PBS) for 24 hours, then removed from water and quickly frozen in liquid nitrogen, and then freeze-dried in a Labconco Freeze Dryer (Kansas City, MO) under vacuum at -50 °C for three days until the samples became completely dry. The freeze-dried hydrogels were fractured to reveal their interior, mounted onto aluminum stubs with double-sided carbon tape, and sputter-coated (Anatech Hummer 6.2 Sputter Coater, Anatech, Union City, CA) with gold for 60 seconds and then visualized using SEM. Pore size was determined manually by measuring the diameter of pores. A minimum of six images were analyzed on each sample. On each SEM image, at least 20 pores were counted and measured and the averaged pore size represents the pore size of each sample.

*In vitro* **degradation**

To determine the effect of neutrophils on the scaffold degradation process, *in vitro* assay using differentiated HL-60 cells (Millius et al., Methods Mol. Biol., vol. 571, pp. 167-177, 2009) was performed. Briefly, HL-60 cells (ATCC, CCL240) were expanded in RPMI1640 medium supplemented with 10% fetal bovine serum (Invitrogen, Carlsbad, CA) at 37°C in a humidified atmosphere of 5% CO2 in air. To induce differentiation, 1.3 % DMSO was added to the culture media. Neutrophil-like cell morphology could be observed within 5 days (Inoue et al., PLoS ONE, vol. 3, no. 8, p. e3068, 2008). To determine degradation kinetics, high ratio dextran hydrogel, low ratio dextran hydrogel, and control scaffolds were incubated with 1 mL of differentiated HL-60 cells (1 x 10$^5$ cells/mL) in differentiation medium. Hydrogel samples were removed from the cultures after 36 and 72 hours, washed with distilled water, and lyophilized in a FreeZone freeze dryer (2.5 L; Labconco, Kansas City, MO) at -48 °C for three days and weighed. The weight loss of the hydrogel degradation comprises both hydrolytic degradation and cell degradation. The extent of biodegradation was estimated from the weight loss of the polymer based on the following equation:

$$\text{Total weight loss} \qquad W_{l,t} = \frac{W_o - W_d}{W_o} \times 100\% \qquad (2)$$

$$\text{Weight loss by cell degradation}\; W_{l,c} = \frac{W_h - W_d}{W_o} \times 100\% \qquad (3)$$

where $W_o$ is the original weight of the hydrogel samples, and $W_d$ is the weight of dry hydrogel samples after being degraded in cell culture, in which the weight loss is attributed to both hydrolysis and cell degradation; while $W_h$ is the weight of dry sample after being degraded by hydrolysis in culture medium (without cell).

**Histology**

Construct explants were collected at days 3, 5, 7, 14, and 21 and fixed using formalin-free fixative (Accustain, Sigma-Aldrich, St. Louis, MO). This fixative was chosen as it preserves both hydrogel structure and endothelial cell immunoreactivity and morphology compared to commonly used formalin based fixatives (Ismail et al., Cardiovasc Pathol, vol. 12, no. 2, pp. 82-90, 2003) or a zinc-based fixative (Hanjaya-Putra D, et al., Blood, vol. 118, no. 3, pp. 804-815, 2011), though this alcoholic based fixative causes swelling of red blood cells (Hanjaya-Putra D, et al., Blood, vol. 118, no. 3, pp. 804-815, 2011). Following fixation of construct explants, samples were dehydrated in graded ethanol (70 to 100 percent), embedded in paraffin, serially sectioned using a microtome (5 $\mu$m), and stained with either hematoxylin and eosin (H&E) or immunohistochemistry for CD31 (Dako, Carpinteria, CA), F4/80 (Invitrogen, Carlsbad, CA), $\alpha$-SMA (Abcam plc, Cambridge, UK) and CD3 (Abcam plc), Von Willebrand Factor (vWF; Dako), Masson's trichrome (Sigma), MPO, VE-cadherin (Abcam plc), and VEGFR2 (Cell Signal Technology, Beverly, MA).

EXAMPLE 2

**Surgery procedure**

The Johns Hopkins University Animal Care and Use Committee approved all procedures. Mice were anesthetized by intraperitoneal injection of ketamine hydrochloride and xylazine hydrochloride; then the dorsum shaved and a depilatory applied (Nair; Church & Dwight Co, Inc., Princeton, NJ). The burn injury was generated as previously reported (Zhang et al., Arch Surg, vol. 145, no. 3, pp. 259-266, 2010). Briefly, a custom-made 220 g aluminum rod was heated in a 100 °C water bath for five minutes. A template (1.2 cm diameter) was used to place the wounds on the posterior -dorsum of each mouse for four seconds. The mice were resuscitated by intraperitoneal injection of saline, using half of the Parkland Formula (4 ml/kg x percent body area), within one hour after burning.

To follow current clinical practice, burn wound excision was performed after 48 hours. Full thickness skin was removed and generated a 8mm in diameter round wound, and covered the wounds with the same size of dextran hydrogels or Integra (control scaffold; both with thickness of ~1mm) and applied DuoDerm dressing. Some wounds were only covered with DuoDerm dressing (dressing-covered control).

**Laser Doppler analysis**

Blood flow in wound areas was measured using a scanning laser Doppler imager (Model LDI2-IR, Moor Instruments, Wilmington, DE) with a near-infrared laser diode at 785nm. The imaging system uses a low-power (2 mW) infrared laser beam to sequentially scan the tissue at several thousand measurement points. For each measurement point, a signal was generated that scales linearly with tissue perfusion, defined as the product of the blood cell velocity and concentration. This signal, termed the *laser Doppler perfusion index* (LDPI), was represented as a two-dimensional color image on a computer screen. The colors produced illustrate the spectrum of perfusion in the wound: dark blue depicts the lowest level of perfusion and red the highest. The system simultaneously produced a photographic image, allowing the direct anatomical comparison of corresponding areas of burn. For each burn, the area of interest was selected by drawing free hand after exporting the image into the software package (the Moor LDI V5.2 software). Then, the mean LDPI value within this area of interest was computed. The scanner was positioned 32 cm above each animal, and scans were performed on day 7 to assess blood flow in the wound margin area. Due to its measurement limits, the Doppler cannot determine the blood flow under either hydrogel or Integra. Thus, only the blood flow in the tissue-scaffold interface was examined in each wound.

**Skin maturity quantification**

The skin structure on day 21 was assessed using H&E-stained histologic sections, according to previously published methods (Ehrbar et al., Circ Res, vol. 94, no. 8, pp. 1124-1132, 2004). At 21 days after the treatment, each wound was assessed histologically using specific criteria for the number of hair follicles, epithelial maturation, and dermal differentiation. For epithelial maturation, the grading was defined according to the following criteria: grade 1, thin and with no reticulation; 2, occasional reticulation; 3, moderate reticulation; 4, thick and with complex reticulation. The grading for dermal differentiation used the following criteria: grade 1, thin, dense, and monotonous fibrosis; 2, thicker but still dense and monotonous fibrosis; 3, two layers but not completely discreet; 4, two discreet layers with superficial fibrosis and loose alveolar tissue within the deep layer. Hair follicles were counted within the wound, between the terminal ends of the panniculus carnosus muscle. Skin thickness was determined by measuring the epidermis, dermis, and fat tissue in H&E- stained histologic sections and Masson's trichrome-stained histologic sections.

EXAMPLE 3

Recent efforts have focused on tailoring the properties of chemically modified dextran hydrogels to promote rapid, functional neovascularization *in vivo.* The incorporation of functional groups - specifically, amine groups - into dextran hydrogel scaffolds was demonstrated to enhance biocompatibility and integration with the host tissue (Sun et al., Journal of Biomedical Materials Research Part A, vol. 93A, no. 3, pp. 1080-1090, 2010). To promote tissue infiltration, neovascularization, and hydrogel degradation, the physical properties of the dextran hydrogels were modified by reducing the degree of substitution of crosslinking groups. This generated a hydrogel, dextran-allyl isocyanate-ethylamine (Dex-AE)/Polyethylene glycol diacrylate (PEGDA) in ratio of 80/20, exhibiting a loose interior architecture but mechanically durable to enable ease of management for transplantation (Sun et al. Biomaterials, vol. 32, no. 1, pp. 95-106, 2011). Stimulating rapid neovascularization through the material-tissue interaction may enhance the burn wound healing process, resulting in skin regeneration. Towards this end, dextran hydrogels can serve as burn wound scaffolds to promote healing **(Figure 2A).** An ideal wound scaffold should protect the wound from bacterial infection, control evaporative water loss and prevent dehydration, allow diffusion of oxygen and carbon dioxide, absorb wound exudate, and enhance healing (Kirker et al., Biomaterials, vol. 23, no. 17, pp. 3661-3671, 2002). For third-degree burn injuries, the wound scaffold should also promote angiogenesis to achieve a favorable healing result. Hence, for viable translational outcomes, dextran hydrogel alone, with no additional growth factors, cytokines, or cells, were considered to determine whether they prove sufficient to treat wound injuries.

A previously established murine burn wound model (Zhang et al., Arch Surg, vol. 145, no. 3, pp. 259-266, 2010; Zhang et al., Wound Repair Regen., vol. 18, no. 2, pp. 193-201, 2010; Light et al., J Burn Care Rehabil, vol. 25, no. 1, pp. 33-44, 2004) was utilized. An important improvement in treating deep burn injuries is to remove badly burned skin followed by the application of wound dressing matrix (i.e., artificial skin), which offers greater protection against wound infection and improves the prognosis of severely burned patients (Schulz et al., Annual Review of Medicine, vol. 51, no. 1, pp. 231-244, 2000). In pursuit of translational outcomes, a procedure for applying dextran hydrogels onto burn wounds in mice was developed; this procedure was designed to follow clinical setting of wound excision 48 hours after the burn. Full-thickness skin was removed from the center of the wound (~8 mm) leaving a small (~2 mm) rim of burned tissue around the excision. The wound was then covered with the same size of hydrogel, and layered with DuoDerm®, an ultra thin dressing, to enable hydrogel placement, protect it from infection, and prevent it from drying **(Figure 9A).** The procedure leaves the hydrogel intact and in place for the entire healing period thus offering opportunities to simplify the management of burn wound treatment. For comparison purposes, Integra®, a cross-linked bovine tendon collagen and glycosaminoglycan matrix, which is the state-of-the-art treatment currently used for patients with deep burns at the Johns Hopkins Burn Center, was applied to some wounds as the control scaffold and others left covered only with dressing.

**Dextran hydrogel promotes wound healing process**

It has been established that if burns primarily heal in less than 21 days, they exhibit minimal scar formation; whereas, if healing remains incomplete by 21 days, a satisfactory scar is unlikely (Cubison et al., Burns, vol. 32, no. 8, pp. 992-999, 2006). The progress in wound healing was analyzed at different time points along the three weeks after treatment application. Significant improvement in the survival of mice with wounds treated with hydrogel and control scaffold was observed compared to mice treated with dressing alone (100% vs 60%, respectively; differences in survival rate between mice treated with hydrogel and controlled scaffolds were not observed). Histological analysis revealed that, compared to wound healing when treated with the control scaffold, dextran hydrogel yielded an accelerated healing kinetics, which resulted in regenerated skin with a defined underlying collagen layer after three weeks of treatment **(Figure 2B; Figure 9B).**

To further understand how the dextran hydrogels promote healing better than control scaffolds, additional burn studies were performed that included two types of amine-modified dextran hydrogels - Dex-AE/ PEGDA in high (80/20) and low (60/40) ratios (Sun et al., Journal of Biomedical Materials Research Part A, vol. 93A, no. 3, pp. 1080-1090, 2010; Sun et al., Biomaterials, vol. 32, no. 1, pp. 95-106, 2011). both high and low ratio dextran hydrogels were found to have a smaller pore size and are softer compared to control scaffolds. Although no significant difference in pore size was observed between the high and low ratio dextran hydrogels, high ratio dextran hydrogel scaffolds are significantly softer than low ratio dextran hydrogels (Figure 3A; Figure 10). From *in vivo* studies (n=6), within 5 days high ratio dextran hydrogel scaffolds (i.e., 80/20) facilitated accelerated cell infiltration compared to low ratio dextran hydrogel scaffolds (Figure 3C). In agreement with previous findings (Sun et al., Biomaterials, vol. 32, no. 1, pp. 95-106, 2011), these results suggest that high ratio dextran hydrogel scaffolds promote rapid wound healing due to their overall physical properties.

**Hydrogel degradation**

After 7 days of treatment, a more fragmented gel structure was observed in the wounds treated with high ratio hydrogel scaffold compared to those treated with low ration hydrogel scaffolds and control scaffold (Figure 4A). As an acute inflammatory response is the first step in third-degree burn wound healing, an *in vitro* degradation study was performed to examine if inflammatory cells have any effect on the degradation. Both types of dextran hydrogel scaffolds and control scaffold cultured with inflammatory cells and their weight loss were measured. To distinguish between scaffold degradation by the cells and hydrolytic degradation process, the scaffolds were also incubated in cell culture media alone. High ratio dextran hydrogel scaffold was degraded more rapidly than low ratio and control scaffold (Figure 4B). Moreover, both cells and hydrolysis accelerate the degradation of the high ratio dextran hydrogel compare to low ratio dextran hydrogel and control scaffold (Figure 4C). The slower degradation of the low ratio dextran hydrogel was attributed to the higher content of nondegradable PEGDA and higher crosslinking density (Sun et al., Journal of Biomedical Materials Research Part A, vol. 93A, no. 3, pp. 1080-1090, 2010). In light of these data, the analysis of burn wound healing kinetics focused on high ratio dextran hydrogel.

**Inflammatory cell infiltration expedites hydrogel degradation**

*In vitro* data show that an efficient neutrophil penetration during the healing process of burn wounds may accelerated the degradation of the hydrogel more than control scaffold. Thus, to determine the contribution of the acute inflammatory response to hydrogel degradation, and the accelerated healing of wounds treated with dextran hydrogels, wounds were analyzed on day 5 following application of the dressing, which is when cellular penetration into the hydrogel was first detected. While differences in T-cell response could not be detected, differences in the accumulation of macrophages and neutrophils was observed between the control scaffold and dextran hydrogel (n=6). As neutrophil recruitment is a normal response in the wound area, more neutrophils were observed on day 7 than on day 5. Following the application of the control scaffold to the wound, neutrophils congregated at the wound periphery by day 5; on day 7, increased neutrophil accumulation at the wound area was observed, generating a thicker layer at the interface of the wound and the treatment area (Figure 5). In the case of dextran hydrogel scaffold, neutrophils infiltrated into the hydrogel scaffolds by day 5 and continued on day 7, resulting in less neutrophil aggregation at the periphery (Figure 5; Figure 11). Indeed, the different response of neutrophils in wounds treated with hydrogels resulted in an almost complete digestion of the hydrogel by day 7 after implantation; however, in the control group, large fragmented sections of the scaffolds remained undigested (as shown above in Figure 4A). These data further confirmed that in addition to hydrolysis, efficient inflammatory cell penetration during the healing process of burn wounds accelerated the degradation of the hydrogel more than control scaffold. The data agree with the results of another study suggesting that neutrophils promoted chitosan hydrogel degradation (Kiyozumi et al., Journal of Biomedical Materials Research Part B: Applied Biomaterials, vol. 79B, no. 1, pp. 129-136, 2006; Khetan et al., Soft Matter, vol. 5, no. 8, pp. 1601-1606, 2009). Additionally, recent studies demonstrated that a degradable hydrogel allowed and directed cell growth *in vitro* (Kloxin et al., Science, vol. 324, no. 5923, pp. 59-63, 2009) compared to non degradable hydrogels. Altogether, a distinctive hydrogel structure, which enables rapid hydrogel degradation, may promote the healing process of third-degree burn wounds by accelerating disintegration of the scaffold during the repair phase.

**Angiogenic cells home to dextran hydrogel**

The angiogenic response was examined, the next step in the burn-healing process. Vascular endothelial growth factor receptor 2 (VEGFR2) is a known marker for endothelial progenitor cells (Peichev et al., Blood, vol. 95, no. 3, pp. 952-958, 2000; Sibal et al., Diabetologia, vol. 52, no. 8, pp. 1464-1473, 2009) and is involved in angiogenic processes (Flamme et al., Developmental Biology, vol. 169, no. 2, pp. 699-712, 1995; Sase et al., J Cell Sci, vol. 122, no. 18, pp. 3303-3311, 2009). In burn wounds treated with hydrogel, cells positive for VEGFR2 and luminal structure formation were detected by day 5. Cells positive for VEGFR2 could not be detected in burn wounds treated with control scaffold **(Figure 6 upper panel)**. Moreover, early vascular networks were observed within the hydrogel area on day 5, as evidenced by positive CD31 staining. These networks expanded and developed by day 7 after implantation in the case of hydrogels but these networks were not observed in the control scaffold -treated wounds **(Figure 6 middle panel)**. Vascular endothelial cadherin (VE-Cad)-positive networks could also be detected on day 7 after hydrogel placement **(Figure 6 lower panel)**. Previous clinical (Fox et al., British Journal of Surgery, vol. 95, no. 2, pp. 244-251, 2008; Gill et al., Circ Res vol., 88, no. 2, pp. 167-174, 2001) and animal model studies (Zhang et al., Arch Surg, vol. 145, no. 3, pp. 259-266, 2010) revealed an increased number of circulating angiogenic cells after burn injuries and found that angiogenesis played a critical role in wound repairs. Results indicate that dextran hydrogels, unlike control scaffolds, accelerated the recruitment of endothelial progenitors and cells to the wound area, enabling rapid neovascularization after a week of treatment.

**Dextran hydrogel promotes angiogenic response**

To better determine the functionality of the developing vasculature, wounds were analyzed on day 7, using laser Doppler to assess blood flow surrounding the wound, and immunohistochemical analysis to quantify the new vascular networks within wounds. The blood flow within the wound area could not be determined, however, because, having covered the wounds with the dressing, they were not accessible to allow accurate measurement by the laser Doppler, and removing the dressing ruptured the healing tissue. Therefore, laser Doppler was performed in the boundary area, as illustrated in **Figure 12A** (n=4). By day 7, dextran hydrogels induced more blood flow to the burn wound area than did the control scaffold and the wound covered with only dressing **(Figure 7A)**. For example, the blood flow with hydrogel was 481 perfusion units, while the blood flow was only 385 perfusion units and 372 perfusion units for dressing-covered controls and control scaffold, respectively **(Figure 7B)**. No significant difference was observed between control scaffold -treated wounds and dressing-covered controls, suggesting that the control scaffold fails to promote angiogenesis in the wound boundary area. To investigate angiogenesis within the wound, Masson's trichrome staining was used, which revealed an increase in delineated vascular networks and the formation of a collagen layer in wounds covered with hydrogels **(Figure 7C)**, further confirmed with specific staining for vascular networks **(Figure 7D)**. Vascular networks stabilized with smooth muscle cells (SMCs) were identified by staining for alpha-smooth muscle actin (a-SMA). Wounds treated with hydrogels demonstrated a significant increase in vascular networks layered with SMCs **(Figure 7E)**. For instance, wounds covered with hydrogel had approximately 714 blood vessels per mm$^2$, while only 271 and 182 blood vessels per mm$^2$ were found for dressing-covered wounds and wounds treated with control scaffold, respectively **(Figure 7F)**. These data support the Doppler analysis findings of increased blood flow around healing wounds treated with hydrogels, demonstrating enhanced vessel growth into the hydrogels compared to control scaffold.

**Dextran hydrogel results in complete skin regeneration**

Finally, the structure of the regenerated skin was analyzed. As mentioned above, healing was observed within three weeks of wound cover. Indeed, at this time point, regression of the vasculature allowed dermal maturation accompanied skin regeneration. The regenerating skin structure was analyzed for epithelial maturation, dermal differentiation, and hair follicles (Ehrbar et al., Circ Res vol. 94, no. 8, pp. 1124-1132, 2004). The results showed that the dextran hydrogel promoted significant skin maturation; hydrogel-treated wounds had a mature epithelial structure with hair follicles and sebaceous glands (n=6) **(Figure 8A-8B; Figure 13)**. Moreover, a significant increase in the number of hair follicles was observed **(Figure 8C)**. Indeed, when the treatment continued for extended periods, hair growth was observed in the center of hydrogel-treated wounds **(Figure 8D)**. In addition, quantification of the skin thickness revealed that the hyperplastic regenerating skin is being remodeled after 3 weeks of treatment, and reaches the thickness of normal mouse skin by 5 weeks of treatment **(Figure 8E; Figure 14)**. Ito et al. demonstrated that nascent follicles arise from epithelial cells outside of the hair follicle stem cell niche, suggesting that epidermal cells in the wound assume a hair follicle stem cell phenotype (Ito et al., Nature, vol. 447, no. 7142, pp. 316-320). Epithelial repair was demonstrated within 14 days of hydrogel application, and mature epithelial morphology with hair follicles and sebaceous glands after 21 days. These results may suggest that the hydrogel facilitate epithelial cell migration or homing to the wound area and support epithelial differentiation.

Functional neovascularization, which facilitates cell and nutrition transportation as well as oxygen exchange, is critical for perfect skin regeneration. In this study, the distinctive hydrogel structure facilitates neutrophil infiltration, neutrophils facilitate hydrogel digestion, and this leads to vascular cell infiltration. Thus, unlike the clinically used scaffold, dextran hydrogels accelerate the recruitment of endothelial cells to the wound area, enabling rapid neovascularization after a week of treatment. The wound treated hydrogel resulted in skin regeneration with appendages (hair follicles and sebaceous glands). Overall, this study clearly demonstrates that dextran hydrogel alone, without the addition of growth factors or cytokines, promotes rapid neovascularization and complete skin regeneration, thus holding great potential to serve as a new device for superior treatment of dermal wounds in clinical applications.

**Claims**

1. A hydrogel for use in promoting skin regeneration comprising topically administering to a subject with an area of a full thickness skin injury damaging the skin, the hydrogel on at least a portion of the injured area, the hydrogel comprising a crosslinked composition comprising: at least 80% of a polysaccharide with at least one monomer having at least one substituted hydroxyl group, wherein the substituted hydroxyl group has the formula (III):

$$-O_1-C(O)NR^7-CH_2CH=CH_2 \qquad (III)$$

wherein $O_1$ is the oxygen atom of said substituted hydroxyl group, $R^7$ is hydrogen or $C_1$-$C_4$ alkyl, wherein the degree of substitution of formula (III) on the polysaccharide is less than 0.2, and wherein the polysaccharide is dextran; and up to 20% of a second crosslinkable molecule, thereby promoting skin regeneration in the injured area.

2. A hydrogel for use in promoting hair follicle regeneration comprising topically administering to a subject with an area of a full thickness skin injury damaging the skin, the hydrogel on the injured area, the hydrogel comprising a crosslinked composition comprising:
at least 80% of a polysaccharide with at least one monomer having at least one substituted hydroxyl group, wherein the substituted hydroxyl group has the formula (III):

$$-O_1-C(O)NR^7-CH_2CH=CH_2 \qquad (III)$$

wherein $O_1$ is the oxygen atom of said substituted hydroxyl group, $R^7$ is hydrogen or $C_1$-$C_4$ alkyl, wherein the degree of substitution of formula (III) on the polysaccharide is less than 0.2, and wherein the polysaccharide is dextran; and up to 20% of a second crosslinkable molecule, thereby promoting hair follicle regeneration.

3. A hydrogel for use in reducing scarring comprising topically administering to a subject with an area of a full thickness skin injury damaging the skin, the_hydrogel on the injured area, the hydrogel comprising a crosslinked composition comprising:
at least 80% of a polysaccharide with at least one monomer having at least one substituted hydroxyl group, wherein the substituted hydroxyl group has the formula (III):

$$-O_1-C(O)NR^7-CH_2CH=CH_2 \qquad (III)$$

wherein $O_1$ is the oxygen atom of said substituted hydroxyl group, $R^7$ is hydrogen or $C_1$-$C_4$ alkyl, wherein the degree of substitution of formula (III) on the polysaccharide is less than 0.2, and wherein the polysaccharide is dextran; and up to 20% of a second crosslinkable molecule, thereby reducing scarring.

4. The hydrogel for use in of any one of claims 1-3, wherein the area of injury damaging the skin is a second degree burn or third degree burn.

5. The hydrogel for use in any one of claims 1-3, wherein said topical administration further comprises placing the hydrogel to cover the entire injured area, and optionally to extend the hydrogel over an uninjured area.

6. The hydrogel for use in any one of claims 1-3, wherein $R^7$ is hydrogen, and the second crosslinkable molecule is poly(ethylene glycol) diacrylate.

7. The hydrogel for use in any one of claims 1-3, wherein the polysaccharide further comprises a second substituted hydroxyl group having the formula (IV), where formula (III) and formula (IV) are different, and the substituted hydroxyl group of formula (III) and formula (IV) may be on the same or different monomers; wherein formula (IV) is

$$Y-(CR^2R^3)_n-Z$$

where Y is $-O_1$- or $-O_1C(O)$-, or $-O_1C(O)NR^1$-, $O_1$ is the oxygen atom of said substituted hydroxyl group, and $R^1$ is hydrogen or $C_1$-$C_4$ alkyl; n = 1, 2, 3, or 4; Z is selected from the group consisting of $-CO_2H$ or $NR^4R^5$, where $R^4$ and $R^5$ are independently hydrogen or $C_1$-$C_4$ alkyl, optionally wherein Z is $NR^4R^5$; $R^2$ and $R^3$ are independently hydrogen,

$C_1$-$C_4$ alkyl, or may combine to form a 3-6 membered ring, and when n>1, $R^2$ and $R^3$ on adjacent carbons may form a double or triple bond, or $R^2$ and $R^3$ on different carbon atoms may form a 3-6 membered ring.

8. The hydrogel for use in any one of claims 1-3, wherein at least one said hydroxyl-substituted saccharide monomer is a glucopyranose monomer.

9. The hydrogel for use in any one of claims 1-3, wherein the dextran has an average molecular weight of at least 20,000.

10. The hydrogel for use in any one of claims 1-3, wherein the second crosslinkable molecule is poly(ethylene glycol) diacrylate, optionally wherein the poly(ethylene glycol) diacrylate has a molecular weight of at least 2000.

11. The hydrogel for use in any one of claims 1-3, further comprising one or more of a protein, oligonucleotide or pharmaceutical agent.

12. The hydrogel for use in claim 11, comprising a protein, wherein the protein is a growth factor, optionally wherein the growth factor is vascular endothelial growth factor (VEGF).

13. The hydrogel for use in claim 11, comprising a pharmaceutical agent, wherein the pharmaceutical agent is an antibiotic, antimicrobial, antibacterial, antifungal, or antiviral compound.

14. The hydrogel for use in any one of claims 1-3, wherein the crosslinked composition does not include a protein or growth factor when topically administered.

15. The hydrogel for use in any of claims 1-3, wherein the crosslinked composition is photocrosslinked.

16. The hydrogel for use in any one of claims 1-3, wherein the injury is excised prior to administration of the hydrogel.

17. The hydrogel for use in of any one of claims 1-3, wherein the injury extends below the deeper layer of the skin to muscle, tendon, or bone.

**Patentansprüche**

1. Hydrogel zur Verwendung zum Fördern von Hautregeneration, umfassend topisches Verabreichen des Hydrogels an ein Subjekt mit einem Bereich einer Hautverletzung der vollen Dicke, die die Haut beschädigt, auf wenigstens einen Teil des verletzten Bereichs, wobei das Hydrogel eine vernetzte Zusammensetzung umfasst, die umfasst: wenigstens 80 % an einem Polysaccharid mit wenigstens einem Monomer mit wenigstens einer substituierten Hydroxygruppe, wobei die substituierte Hydroxygruppe die Formel (III) aufweist:

$$-O_1-C(O)NR^7-CH_2CH=CH_2 \qquad (III),$$

wobei $O_1$ das Sauerstoffatom der substituierten Hydroxygruppe ist, $R^7$ Wasserstoff oder $C_1$-$C_4$Alkyl ist, wobei der Grad der Substitution der Formel (III) an dem Polysaccharid kleiner als 0,2 ist und wobei das Polysaccharid Dextran ist; und bis zu 20 % an einem zweiten vernetzbaren Molekül, um dadurch Hautregeneration in dem verletzten Bereich zu fördern.

2. Hydrogel zur Verwendung zum Fördern von Haarfollikelregeneration, umfassend topisches Verabreichen des Hydrogels an ein Subjekt mit einem Bereich einer Hautverletzung der vollen Dicke, die die Haut beschädigt, auf den verletzten Bereich, wobei das Hydrogel eine vernetzte Zusammensetzung umfasst, die umfasst: wenigstens 80 % an einem Polysaccharid mit wenigstens einem Monomer mit wenigstens einer substituierten Hydroxygruppe, wobei die substituierte Hydroxygruppe die Formel (III) aufweist:

$$-O_1-C(O)NR^7-CH_2CH=CH_2 \qquad (III),$$

wobei $O_1$ das Sauerstoffatom der substituierten Hydroxygruppe ist, $R^7$ Wasserstoff oder $C_1$-$C_4$Alkyl ist, wobei der Grad der Substitution der Formel (III) an dem Polysaccharid kleiner als 0,2 ist und wobei das Polysaccharid Dextran ist; und bis zu 20 % an einem zweiten vernetzbaren Molekül, um dadurch Haarfollikelregeneration zu fördern.

3. Hydrogel zur Verwendung zum Verringern von Narbenbildung, umfassend topisches Verabreichen des Hydrogels an ein Subjekt mit einem Bereich einer Hautverletzung der vollen Dicke, die die Haut beschädigt, auf den verletzten Bereich, wobei das Hydrogel eine vernetzte Zusammensetzung umfasst, die umfasst:

wenigstens 80 % an einem Polysaccharid mit wenigstens einem Monomer mit wenigstens einer substituierten Hydroxygruppe, wobei die substituierte Hydroxygruppe die Formel (III) aufweist:

$$-O_1-C(O)NR^7-CH_2CH=CH_2 \qquad (III),$$

wobei $O_1$ das Sauerstoffatom der substituierten Hydroxygruppe ist, $R^7$ Wasserstoff oder $C_1$-$C_4$Alkyl ist, wobei der Grad der Substitution der Formel (III) an dem Polysaccharid kleiner als 0,2 ist und wobei das Polysaccharid Dextran ist; und bis zu 20 % an einem zweiten vernetzbaren Molekül, um dadurch Narbenbildung zu verringern.

4. Hydrogel zur Verwendung gemäß einem der Ansprüche 1-3, wobei der Bereich der Verletzung, die die Haut beschädigt, eine Verbrennung zweiten Grades oder eine Verbrennung dritten Grades ist.

5. Hydrogel zur Verwendung gemäß einem der Ansprüche 1-3, wobei die topische Verabreichung ferner umfasst, das Hydrogel so zu platzieren, dass es den gesamten verletzten Bereich bedeckt, und gegebenenfalls das Hydrogel über einen nicht verletzten Bereich auszudehnen.

6. Hydrogel zur Verwendung gemäß einem der Ansprüche 1-3, wobei $R^7$ Wasserstoff ist und das zweite vernetzbare Molekül Poly(ethylenglycol)diacrylat ist.

7. Hydrogel zur Verwendung gemäß einem der Ansprüche 1-3, wobei das Polysaccharid ferner eine zweite substituierte Hydroxygruppe mit der Formel (IV) umfasst, wobei die Formel (III) und die Formel (IV) verschieden sind, und die substituierte Hydroxygruppe der Formel (III) und der Formel (IV) an den gleichen oder verschiedenen Monomeren vorliegen können; wobei die Formel (IV)

$$Y-(CR^2R^3)_n-Z$$

ist, wobei Y $-O_1-$ oder $-O_1C(O)-$ oder $-O_1C(O)NR^1-$ ist, $O_1$ das Sauerstoffatom der substituierten Hydroxygruppe ist und $R^1$ Wasserstoff oder $C_1$-$C_4$Alkyl ist; n = 1, 2, 3 oder 4; Z ausgewählt ist aus der Gruppe bestehend aus $-CO_2H$ und $NR^4R^5$, wobei $R^4$ und $R^5$ unabhängig Wasserstoff oder $C_1$-$C_4$Alkyl sind, wobei Z gegebenenfalls $NR^4R^5$ ist; $R^2$ und $R^3$ unabhängig Wasserstoff oder $C_1$-$C_4$Alkyl sind oder kombiniert sein können, um einen 3-6-gliedrigen Ring zu bilden, und wenn n > 1, $R^2$ und $R^3$ an benachbarten Kohlenstoffen eine Doppel- oder Dreifachbindung bilden können oder $R^2$ und $R^3$ an verschiedenen Kohlenstoffen einen 3-6-gliedrigen Ring bilden können.

8. Hydrogel zur Verwendung gemäß einem der Ansprüche 1-3, wobei wenigstens eines der hydroxysubstituierten Saccharidmonomere ein Glucopyranosemonomer ist.

9. Hydrogel zur Verwendung gemäß einem der Ansprüche 1-3, wobei das Dextran ein mittleres Molekulargewicht von wenigstens 20.000 aufweist.

10. Hydrogel zur Verwendung gemäß einem der Ansprüche 1-3, wobei das zweite vernetzbare Molekül Poly(ethylenglycol)diacrylat ist, wobei das Poly(ethylenglycol)diacrylat gegebenenfalls ein Molekulargewicht von wenigstens 2000 aufweist.

11. Hydrogel zur Verwendung gemäß einem der Ansprüche 1-3, ferner umfassend eines oder mehrere von einem Protein, Oligonucleotid oder pharmazeutischen Mittel.

12. Hydrogel zur Verwendung gemäß Anspruch 11, umfassend ein Protein, wobei das Protein ein Wachstumsfaktor ist, wobei der Wachstumsfaktor gegebenenfalls vaskulärer endothelialer Wachstumsfaktor (VEGF) ist.

13. Hydrogel zur Verwendung gemäß Anspruch 11, umfassend ein pharmazeutisches Mittel, wobei das pharmazeutische Mittel eine antibiotische, antimikrobelle, antibakterielle, antifungielle oder antivirale Verbindung ist.

14. Hydrogel zur Verwendung gemäß einem der Ansprüche 1-3, wobei die vernetzte Zusammensetzung, wenn topisch verabreicht, kein/keinen Protein oder Wachstumsfaktor enthält.

**15.** Hydrogel zur Verwendung gemäß einem der Ansprüche 1-3, wobei die vernetzte Zusammensetzung photovernetzt ist.

**16.** Hydrogel zur Verwendung gemäß einem der Ansprüche 1-3, wobei die Verletzung vor der Verabreichung des Hydrogels exzidiert wird.

**17.** Hydrogel zur Verwendung gemäß einem der Ansprüche 1-3, wobei sich die Verletzung unter die tiefere Schicht der Haut auf Muskel, Sehne oder Knochen erstreckt.

**Revendications**

**1.** Hydrogel pour une utilisation destinée à favoriser la régénération de la peau, comprenant l'administration par voie topique à un sujet ayant une zone de lésion d'épaisseur totale de la peau et endommageant celle-ci, de l'hydrogel sur au moins une portion de la zone lésée, l'hydrogel comprenant une composition réticulée comprenant :
au moins 80 % d'un polysaccharide avec au moins un monomère ayant au moins un groupement hydroxyle substitué, où le groupement hydroxyle substitué répond à la formule (III) :

$$-O_1\text{-}C(O)NR^7\text{-}CH_2CH{=}CH_2 \qquad (III)$$

où $O_1$ est l'atome d'oxygène dudit groupement hydroxyle substitué, $R^7$ est hydrogène ou $C_1$-$C_4$-alkyle, où le degré de substitution de formule (III) sur le polysaccharide est inférieur à 0,2, et où le polysaccharide est le dextrane ; et jusqu'à 20 % d'une deuxième molécule réticulable, favorisant ainsi la régénération de la peau dans la zone lésée.

**2.** Hydrogel pour une utilisation destinée à favoriser la régénération des follicules capillaires, comprenant l'administration par voie topique à un sujet ayant une zone de lésion d'épaisseur totale de la peau et endommageant celle-ci, de l'hydrogel sur la zone lésée, l'hydrogel comprenant une composition réticulée comprenant :
au moins 80 % d'un polysaccharide avec au moins un monomère ayant au moins un groupement hydroxyle substitué, où le groupement hydroxyle substitué répond à la formule (III) :

$$-O_1\text{-}C(O)NR^7\text{-}CH_2CH{=}CH_2 \qquad (III)$$

où $O_1$ est l'atome d'oxygène dudit groupement hydroxyle substitué, $R^7$ est hydrogène ou $C_1$-$C_4$-alkyle, où le degré de substitution de formule (III) sur le polysaccharide est inférieur à 0,2, et où le polysaccharide est le dextrane ; et jusqu'à 20 % d'une deuxième molécule réticulable, favorisant ainsi la régénération des follicules capillaires.

**3.** Hydrogel pour une utilisation destinée à réduire les cicatrices, comprenant l'administration par voie topique à un sujet ayant une zone de lésion d'épaisseur totale de la peau et endommageant celle-ci, de l'hydrogel sur la zone lésée, l'hydrogel comprenant une composition réticulée comprenant :
au moins 80 % d'un polysaccharide avec au moins un monomère ayant au moins un groupement hydroxyle substitué, où le groupement hydroxyle substitué répond à la formule (III) :

$$-O_1\text{-}C(O)NR^7\text{-}CH_2CH{=}CH_2 \qquad (III)$$

où $O_1$ est l'atome d'oxygène dudit groupement hydroxyle substitué, $R^7$ est hydrogène ou $C_1$-$C_4$-alkyle, où le degré de substitution de formule (III) sur le polysaccharide est inférieur à 0,2, et où le polysaccharide est le dextrane ; et jusqu'à 20 % d'une deuxième molécule réticulable, réduisant ainsi les cicatrices.

**4.** Hydrogel pour une utilisation selon l'une quelconque des revendications 1-3, dans lequel la zone de lésion endommageant la peau est une brûlure au deuxième degré ou une brûlure au troisième degré.

**5.** Hydrogel pour une utilisation selon l'une quelconque des revendications 1-3, dans lequel ladite administration topique comprend en outre le placement de l'hydrogel de façon à recouvrir l'ensemble de la zone lésée, et éventuellement pour étendre l'hydrogel sur une zone non lésée.

**6.** Hydrogel pour une utilisation selon l'une quelconque des revendications 1-3, dans lequel $R^7$ est hydrogène, et la deuxième molécule réticulable est le diacrylate de poly(éthylène glycol).

**7.** Hydrogel pour une utilisation selon l'une quelconque des revendications 1-3, dans lequel le polysaccharide comprend en outre un deuxième groupement hydroxyle substitué répondant à la formule (IV), où la formule (III) et la formule (IV) sont différentes, et le groupement hydroxyle substitué de formule (III) et de formule (IV) peut se trouver sur des monomères identiques ou différents ; où la formule (IV) est

$$Y\text{-}(CR^2R^3)_n\text{-}Z$$

où Y est $-O_1-$ ou $-O_1C(O)-$ ou $-O_1C(O)NR^1-$, $O_1$ est l'atome d'oxygène dudit groupement hydroxyle substitué, et $R^1$ est hydrogène ou $C_1$-$C_4$-alkyle ; n= 1, 2, 3 ou 4 ; Z est choisi dans le groupe constitué par $-CO_2H$ ou $NR^4R^5$, où $R^4$ et $R^5$ sont indépendamment hydrogène ou $C_1$-$C_4$-alkyle, éventuellement où Z est $NR^4R^5$ ; $R^2$ et $R^3$ sont indépendamment hydrogène, $C_1$-$C_4$-alkyle, ou peuvent être combinés pour former un cycle de 3-6 chaînons, et lorsque n>1, $R^2$ et $R^3$ sur des carbones adjacents peuvent former une liaison double ou triple, ou $R^2$ et $R^3$ sur des atomes de carbone différents peuvent former un cycle de 3-6 chaînons.

**8.** Hydrogel pour une utilisation selon l'une quelconque des revendications 1-3, dans lequel au moins l'un desdits monomères saccharidiques substitués par hydroxyle est un monomère de glucopyranose.

**9.** Hydrogel pour une utilisation selon l'une quelconque des revendications 1-3, dans lequel le dextrane possède un poids moléculaire moyen d'au moins 20 000.

**10.** Hydrogel pour une utilisation selon l'une quelconque des revendications 1-3, dans lequel la deuxième molécule réticulable est le diacrylate de poly(éthylène glycol), éventuellement où le diacrylate de poly(éthylène glycol) possède un poids moléculaire d'au moins 2000.

**11.** Hydrogel pour une utilisation selon l'une quelconque des revendications 1-3, comprenant en outre un(e) ou plusieurs parmi une protéine, un oligonucléotide ou un agent pharmaceutique.

**12.** Hydrogel pour une utilisation selon la revendication 11, comprenant une protéine, où la protéine est un facteur de croissance, éventuellement où le facteur de croissance est le facteur de croissance endothélial vasculaire (VEGF).

**13.** Hydrogel pour une utilisation selon la revendication 11, comprenant un agent pharmaceutique, où l'agent pharmaceutique est un composé antibiotique, antimicrobien, antibactérien, antifongique, ou antiviral.

**14.** Hydrogel pour une utilisation selon l'une quelconque des revendications 1-3, dans lequel la composition réticulée ne comporte pas de protéine ou de facteur de croissance lorsqu'elle est administrée par voie topique.

**15.** Hydrogel pour une utilisation selon l'une quelconque des revendications 1-3, dans lequel la composition réticulée est photo-réticulée.

**16.** Hydrogel pour une utilisation selon l'une quelconque des revendications 1-3, dans lequel la lésion est excisée préalablement à l'administration de l'hydrogel.

**17.** Hydrogel pour une utilisation selon l'une quelconque des revendications 1-3, dans lequel la lésion s'étend en dessous de la couche la plus profonde de la peau jusqu'au muscle, au tendon ou à l'os.

Dex-AI

$R = -OCNHCH_2CH=CH_2$

PEGDA

Irgacure 2959
UV photocrosslinking

**Figure 1**

(Dex-AE)

(PEGDA)

UV

**Crosslinked Hydrogel**

Apply hydrogel
on 3rd degree burn

**Burn Wound**

**Neovascularization &
Skin Regeneration**

**Figure 2A**

Figure 2B

EP 2 704 727 B1

Figure 3

**4A**

Control          60/40          80/20

Day 5

Day 7

## Figure 4

EP 2 704 727 B1

Figure 4, cont.

**Figure 5**

Figure 6

Figure 7

Figure 7, cont.

EP 2 704 727 B1

Figure 7, cont.

Figure 8

EP 2 704 727 B1

8D

Photos

H&E

**Figure 8, cont.**

Figure 8, cont.

**9A**

1. Burn (1.2cm)  2. Wound Excision  3. Apply hydrogel  4. Cover to protect

Dressing  Control  Hydrogel

**9B**

**Figure 9**

Figure 10

MPO F4/80 CD3

Day 5

Day 7

**Figure 11**

12A

12B

**Figure 12**

Figure 13

Figure 14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61483518
- US 61563954
- WO 2010078036 A
- WO 2012003370 A
- WO 2011060095 A

**Non-patent literature cited in the description**

- **ZHANG et al.** *Arch Surg,* 2010, vol. 145 (3), 259-266
- **SEN et al.** *J. Burn Care Res.,* 2010, vol. 31 (6), 836-848
- **FAGENHOLZ et al.** *J Burn Care Res,* 2007, vol. 28 (5), 681-690
- **ZHANG et al.** *Wound Repair Regen.,* 2010, vol. 18 (2), 193-201
- **KIRKER et al.** *Biomaterials,* 2002, vol. 23 (17), 3661-3671
- **GURTNER et al.** *Nature,* 2008, vol. 453 (7193), 314-321
- **LI et al.** *Microscopy Research and Technique,* 2003, vol. 60 (1), 107-114
- **MARTIN.** *Science,* 1997, vol. 276 (5309), 75-81
- **TIBBS.** *Radiotherapy and Oncology,* 1997, vol. 42 (2), 99-106
- **HAROON et al.** *The FASEB Journal,* 1999, vol. 13 (13), 1787-1795
- **STEED.** *Surgical Clinics of North America,* 1997, vol. 77 (3), 575-586
- **KIM et al.** *J Invest Dermatol,* 2008, vol. 128 (7), 1812-1820
- **GREENHALGH.** *The International Journal of Biochemistry & Cell Biology,* 1998, vol. 30 (9), 1019-1030
- **SINGER et al.** *New England Journal of Medicine,* 1999, vol. 341 (10), 738-746
- **TREDGET.** *Journal of Trauma-Injury Infection and Critical Care,* 2007, vol. 62 (6), S69-S69
- **BOUCARD et al.** *Biomaterials,* 2007, vol. 28 (24), 3478-3488
- **KIYOZUMI et al.** *Burns,* 2007, vol. 33 (5), 642-648
- **KIM et al.** *Biomaterials,* 2009, vol. 30 (22), 3742-3748
- **MADSEN et al.** *Biomacromolecules,* 2008, vol. 9 (8), 2265-2275
- **SHEPHERD et al.** *Biomaterials,* 2011, vol. 32 (1), 258-267
- **BALAKRISHNAN et al.** *Biomaterials,* 2005, vol. 26 (32), 6335-6342
- **PUOLAKKAINEN et al.** *Journal of Surgical Research,* 1995, vol. 58 (3), 321-329
- **KIYOZUMI et al.** *Journal of Biomedical Materials Research Part B: Applied Biomaterials,* 2006, vol. 79B (1), 129-136
- **LIU et al.** *Biomaterials,* 2009, vol. 30 (8), 1453-1461
- **LEE et al.** *Mol Ther,* 2007, vol. 15 (6), 1189-1194
- **ZHANG et al.** *Biomaterials,* 2002, vol. 23, 2641-2648
- **MUSLIM et al.** *Carbohydr. Polym.,* 2001, vol. 46, 323-330
- **HU et al.** *Int. J. Biochem. Cell. Biol.,* 2002, vol. 34, 396-402
- **SUN et al.** *Journal of Biomedical Materials Research Part A,* 2010, vol. 93A (3), 1080-1090
- **SUN et al.** *Biomaterials,* 2011, vol. 32 (1), 95-106
- **MILLIUS et al.** *Methods Mol. Biol.,* 2009, vol. 571, 167-177
- **INOUE et al.** *PLoS ONE,* 2008, vol. 3 (8), e3068
- **ISMAIL et al.** *Cardiovasc Pathol,* 2003, vol. 12 (2), 82-90
- **HANJAYA-PUTRA D et al.** *Blood,* 2011, vol. 118 (3), 804-815
- **EHRBAR et al.** *Circ Res,* 2004, vol. 94 (8), 1124-1132
- **LIGHT et al.** *J Burn Care Rehabil,* 2004, vol. 25 (1), 33-44
- **SCHULZ et al.** *Annual Review of Medicine,* 2000, vol. 51 (1), 231-244
- **CUBISON et al.** *Burns,* 2006, vol. 32 (8), 992-999
- **KHETAN et al.** *Soft Matter,* 2009, vol. 5 (8), 1601-1606
- **KLOXIN et al.** *Science,* 2009, vol. 324 (5923), 59-63

- **PEICHEV et al.** *Blood,* 2000, vol. 95 (3), 952-958
- **SIBAL et al.** *Diabetologia,* 2009, vol. 52 (8), 1464-1473
- **FLAMME et al.** *Developmental Biology,* 1995, vol. 169 (2), 699-712
- **SASE et al.** *J Cell Sci,* 2009, vol. 122 (18), 3303-3311
- **FOX et al.** *British Journal of Surgery,* 2008, vol. 95 (2), 244-251
- **GILL et al.** *Circ Res,* 2001, vol. 88 (2), 167-174
- **ITO et al.** *Nature,* vol. 447 (7142), 316-320